# EUROPEAN PATENT SPECIFICATION

(11) **EP 1 165 553 B1**
(45) Date of publication and mention of the grant of the patent: **03.06.2009**
(21) Application number: 01906971.5
(22) Date of filing: 02.02.2001
(51) Int. Cl.: C07D 409/12, A61K 31/335, A61P 35/00

(54) **C10 ESTER SUBSTITUTED TAXANES AS ANTITUMOR AGENTS**
C10-TAXANESTER ALS ANTITUMORMITTEL
TAXANES SUBSTITUES PAR UN ESTER EN C10 COMME AGENTS ANTITUMORAUX

(30) Priority: 02.02.2000 US 179782 P
(43) Date of publication of application: 02.01.2002
(73) Proprietor: Florida State University Research Foundation, Inc., Tallahassee, Florida 32306-2763 (US)
(72) Inventor: HOLTON, Robert A. c/o Florida State University, Tallahassee, FL 32306-2763 (US)
(74) Representative: Maiwald Patentanwalts GmbH
(86) International application number: PCT/US2001/003623
(87) International publication number: WO 2001/057032

(56) References cited:
- EP-A- 0 534 709
- WO-A-00/53592
- WO-A-96/13495
- WO-A-97/32578
- WO-A-97/44026
- WO-A-97/44063
- US-A- 5 243 045
- US-A- 5 250 683
- I. OJIMA ET AL.: "Synthesis and structure-activity relationships of new second-generation taxoids" BIOORG. MED. CHEM. LETT., vol. 9, no. 24, 1999, pages 3423-3428, XP004185527
- I. OJIMA ET AL.: "Synthesis and biological activity of novel 3'-trifluoromethyl taxoids" BIOORG. MED. CHEM. LETT., vol. 7, no. 2, 1997, pages 133-138, XP004135981
- I. OJIMA ET AL.: "Enantiopure fluorine-containing taxoids: potent anticancer agents and versatile probes for biomedical problems" J. FLUORINE CHEM., vol. 97, no. 1-2, 1999, pages 3-10, XP004172067
- J. KANT ET AL.: "A Chemoselective Approach to Functionalize the C-10 Position of 10-Deacetylbaccatin III. Synthesis and Biological Properties of Novel C-10 Taxol (R) Analogues" TETRAHEDRON LETT., vol. 35, no. 31, 1994, pages 5543-5546, XP002915065
- J. DUBOIS ET AL.: "Fluorescent and Biotinylated Analogues of Docetaxel: Synthesis and Biological Evaluation" BIOORG. MED. CHEM. LETT., vol. 3, no. 10, 1995, pages 1357-1368, XP000999888
- B.-X. SHI ET AL.: "Sudies on the Quantitative Structure-activity Relationships of Paclitaxel Analogues" GAODENG XUEXIAO HUAXUE XUEBAO, vol. 21, no. 3, 2000, pages 401-406, XP000999959

## Description

### BACKGROUND OF THE INVENTION

The present invention is directed to novel taxanes which have exceptional utility as antitumor agents.

The taxane family of terpenes, of which baccatin III and taxol are members, has been the subject of considerable interest in both the biological and chemical arts. Taxol itself is employed as a cancer chemotherapeutic agent and possesses a broad range of tumor-inhibiting activity. Taxol has a 2'R, 3'S configuration and the following structural formula: 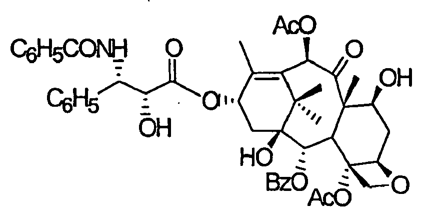 wherein Ac is acetyl.

Colin et al. reported in U.S. Patent 4,814,470 that certain taxol analogs have an activity significantly greater than that of taxol. One of these analogs, commonly referred to as docetaxel, has the following structural formula:

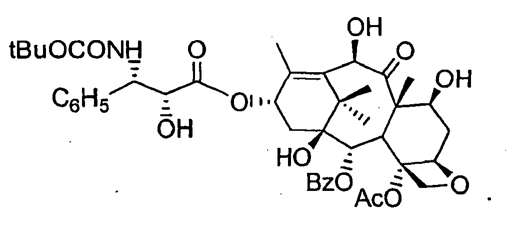

I. Ojima et al.; Bioorg. Med. Chem. Lett., 1999, 9, 3423-3428 describes a series of second-generation taxoids bearing a substitutent on the C-2- benzoyl group and modifications at C-3'/C-10 positions. I. Ojima et al.; Bioorg. Med. Chem. Lett., 1997, 7, 133-138 describes second generation taxoids possessing a trifluoromethyl moiety in place of the 3'-phenyl group. I. Ojima et al.; J. Fluorine Chem. 1999, 97, 3-10 describes taxoids bearing trifluoromethyl or difluoromethyl groups at the C-3' position. J. Dubois et al.; Bioorg. Med. Chem. Lett., 1995, 3, 1357-1368 describes certain docetaxol analogues. J. Kant et al.; Tetrahedron Lett. 1994, 35, 5543-5546 describes a chemoselective approach to functionalize the C-10 position of 10-deacetyl baccatin III. WO 97/44026 and WO 97/44063 describe taxanes that are conjugates of *cis-*docosahexaenoic acid and taxotere. WO 97/32578 describes new taxoids useful as antitumor agents or their precursors. WO 96/13495 describes taxoid derivatives, their preparation and their use as antitumor agents. Taxanes valuable as antileukemia and antitumor agents are described in U.S. Patent No. 5,243,045, U.S. Patent No. 5,250,683 and European Patent Application No. 0534 709 Al. None of these documents discloses taxane derivatives having heterocyclo derivatives at the C₃'-position. U.S. Patent No. 6,268,381 describes taxane derivatives having piperazine and piperidine groups at the C-10 position.

Although taxol and docetaxel are useful chemotherapeutic agents, there are limitations on their effectiveness, including limited efficacy against certain types of cancers and toxicity to subjects when administered at various doses. Accordingly, a need remains for additional chemotherapeutic agents with improved efficacy and less toxicity.

### SUMMARY OF THE INVENTION

Among the objects of the present invention, therefore, is the provision of taxanes which compare favorably to taxol and docetaxel with respect to efficacy as anti-tumor agents and with respect to toxicity. In general, these taxanes possess an ester substituent other than formate, acetate and heterosubstituted acetate at C-10, a hydroxy substituent at C-7 and a range of C-3' substituents.

Briefly, therefore, the present invention is directed to the taxane composition, per se, to pharmaceutical compositions comprising the taxane and a pharmaceutically acceptable carrier, and to methods of administration.

Other objects and features of this invention will be in part apparent and in part pointed out hereinafter.

### DETAILED DESCRIPTION OF THE PREFERRED EMBODIMENTS

In one embodiment of the present invention, the taxanes of the present invention correspond to structure (1): 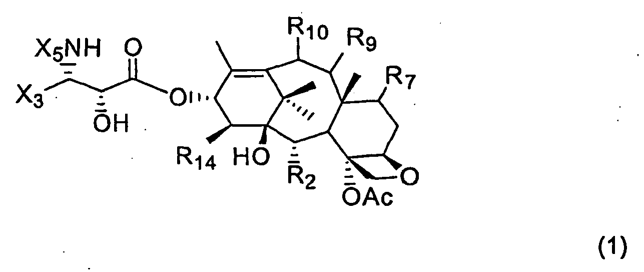 wherein
R₂ is acyloxy;
R₇ is hyd roxy;
R₉ is keto, hydroxy, or acyloxy;
R₁₀ is R₁₀ₐ,COO-;
R₁₀ₐ is hydrocarbyl, or substituted hydrocarbyl, wherein said hydrocarbyl or substituted hydrocarbyl contains carbon atoms in the alpha and beta positions relative to the carbon of which R₁₀ₐ is a substituent;
R₁₄ is hydrogen or hydroxy;
X₃ is substituted or unsubstituted heterocyclo;
X₅ is -COX₁₀, -COOX₁₀, or -CONHX₁₀;
X₁₀ is hydrocarbyl, substituted hydrocarbyl, or heterocyclo;
Ac is acetyl; and
R₇, R₉, and R₁₀ independently have the alpha or beta stereochemical configuration.

In one embodiment, R₂ is an ester (R₂ₐC(O)O-), a carbamate (R₂ₐR_{2b}NC(O)O-), a carbonate (R₂ₐOC(O)O-), or a thiocarbamate (R₂ₐSC(O)O-) wherein R₂ₐ and R_{2b} are independently hydrogen, hydrocarbyl, substituted hydrocarbyl or heterocyclo. In a preferred embodiment, R₂ is an ester (R₂ₐC(O)O-), wherein R₂ₐ is aryl or heteroaromatic. In another preferred embodiment, R₂ is an ester (R₂ₐC(O)O-), wherein R₂ₐ is substituted or unsubstituted phenyl, furyl, thienyl, or pyridyl. In one particularly preferred embodiment, R₂ is benzoyloxy.

While R₉ is keto in one embodiment of the present invention, in other embodiments R₉ may have the alpha or beta stereochemical configuration, preferably the beta stereochemical configuration, and may be, for example, α- or β-hydroxy, or α- or β-acyloxy. For example, when R₉ is acyloxy, it may be an ester (R₉ₐC(O)O-), a carbamate (R₉ₐR_{9b}NC(O)O-), a carbonate (R₉ₐOC(O)O-), or a thiocarbamate (R₉ₐSC(O)O-) wherein R₉ₐ and R_{9b} are independently hydrogen, hydrocarbyl, substituted hydrocarbyl or heterocyclo. If R₉ is an ester (R₉ₐC(O)O-), R₉ₐ is substituted or unsubstituted alkyl, substituted or unsubstituted alkenyl, substituted or unsubstituted aryl or substituted or unsubstituted heteroaromatic. Still more preferably, R₉ is an ester (R₉ₐC(O)O-), wherein R₉ₐ is substituted or unsubstituted phenyl, substituted or unsubstituted furyl, substituted or unsubstituted thienyl, or substituted or unsubstituted pyridyl. In one embodiment R₉ is (R₉ₐC(O)O-) wherein R₉ₐ is methyl, ethyl, propyl (straight, branched or cyclic), butyl (straight, branched or cyclic), pentyl, (straight, branched or cyclic), or hexyl (straight, branched or cyclic). In another embodiment R₉ is (R₉ₐC(O)O-) wherein R₉ₐ is substituted methyl, substituted ethyl, substituted propyl (straight, branched or cyclic), substituted butyl (straight, branched or cyclic), substituted pentyl, (straight, branched or cyclic)), or substituted hexyl (straight, branched or cyclic) wherein the substituent(s) is/are selected from the group consisting of heterocyclo, alkoxy, alkenoxy, alkynoxy, aryloxy, hydroxy, protected hydroxy, keto, acyloxy, nitro, amino, amido, thiol, ketal, acetal, ester and ether moieties, but not phosphorous containing moieties.

In one embodiment, R₁₀ is R₁₀ₐCOO- wherein R₁₀ₐ is (i) substituted or unsubstituted C₂ to C₈ alkyl (straight, branched or cyclic), such as ethyl, propyl, butyl, pentyl, or hexyl; (ii) substituted or unsubstituted C₂ to C₈ alkenyl (straight, branched or cyclic), such as ethenyl, propenyl, butenyl, pentenyl or hexenyl; (iii) substituted or unsubstituted C₂ to C₈ alkynyl (straight or branched) such as ethynyl, propynyl, butynyl, pentynyl, or hexynyl; (iv) substituted or unsubstituted phenyl. The substituents may be hydrocarbyl or any of the heteroatom containing substituents identified elsewhere herein for substituted hydrocarbyl. In a preferred embodiment, R₁₀ₐ is ethyl, straight, branched or cyclic propyl, straight, branched or cyclic butyl, straight, branched or cyclic pentyl, straight, branched or cyclic hexyl, straight or branched propenyl, or isobutenyl. In another embodiment, R₁₀ₐ is substituted ethyl, substituted propyl (straight, branched or cyclic), substituted propenyl (straight or branched), or substituted isobutenyl, wherein the substituent(s) is/are selected from the group consisting of heterocyclo, alkoxy, alkenoxy, alkynoxy, aryloxy, hydroxy, protected hydroxy, keto, acyloxy, nitro, amino, amido, thiol, ketal, acetal, ester and ether moieties, but not phosphorous containing moieties.

Exemplary X₃ substituents include substituted or unsubstituted heteroaromatic containing 5 or 6 ring atoms. Exemplary preferred X₃ substituents include furyl, thienyl, and pyridyl.

Exemplary X₅ substituents include -COX₁₀, -COOX₁₀ or -CONHX₁₀ wherein X₁₀ is substituted or unsubstituted alkyl, alkenyl, phenyl or heteroaromatic. Exemplary preferred X₅ substituents include -COX_{10'} -COOX₁₀ or -CONHX₁₀ wherein X₁₀ is (i) substituted or unsubstituted C₁ to C₈ alkyl such as substituted or unsubstituted ethyl, ethyl, propyl (straight, branched or cyclic), butyl (straight, branched or cyclic), pentyl (straight, branched or cyclic), or hexyl (straight, branched or cyclic); (ii) substituted or unsubstituted C₂ to C₈ alkenyl such as substituted or unsubstituted ethenyl, propenyl (straight, branched or cyclic), butenyl (straight, branched or cyclic), pentenyl (straight, branched or cyclic) or hexenyl (straight, branched or cyclic); (iii) substituted or unsubstituted C₂ to C₈ alkynyl such as substituted or unsubstituted ethynyl, propynyl (straight or branched), butynyl (straight or branched), pentynyl (straight or branched), or hexynyl (straight or branched); (iv) substituted or unsubstituted phenyl, or (v) substituted or unsubstituted heteroaromatic such as furyl, thienyl, or pyridyl, wherein the substituent(s) is/are selected from the group consisting of heterocyclo, alkoxy, alkenoxy, alkynoxy, aryloxy, hydroxy, protected hydroxy, keto, acyloxy, nitro, amino, amido, thiol, ketal, acetal, ester and ether moieties, but not phosphorous containing moieties.

In one embodiment of the present invention, the taxanes of the present invention correspond to structure (2): 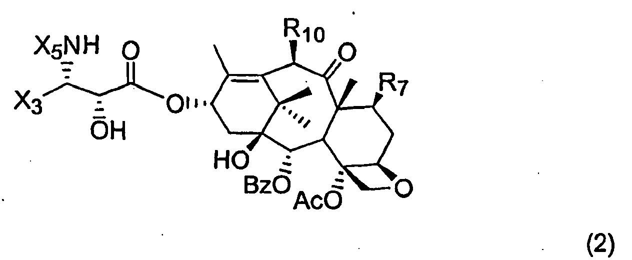 wherein
R₇ is hydroxy;
R₁₀ is R₁₀ₐCOO-;
X₃ is substituted or unsubstituted heterocyclo;
X₅ is -COX₁₀, -COOX₁₀, or -CONHX₁₀; and
X₁₀ is hydrocarbyl, substituted hydrocarbyl, or heterocyclo; and
R₁₀ₐ is hydrocarbyl, or substituted hydrocarbyl, wherein said hydrocarbyl or substituted hydrocarbyl contains carbon atoms in the alpha and beta positions relative to the carbon of which R₁₀ₐ is a substituent;
Bz is benzoyl; and
Ac is acetyl.
For example, in this preferred embodiment in which the taxane corresponds to structure (2), R₁₀ₐ may be substituted or unsubstituted ethyl, propyl or butyl, more preferably substituted or unsubstituted ethyl or propyl, still more preferably substituted or unsubstituted ethyl, and still more preferably unsubstituted ethyl. While R₁₀ₐ is selected from among these, in one embodiment X₃ is selected from substituted or unsubstituted heterocyclo, and still more preferably heterocyclo such as furyl, thienyl or pyridyl. While R₁₀ₐ and X₃ are selected from among these, in one embodiment X₅ is selected from -COX₁₀ wherein X₁₀ is phenyl, alkyl or heterocyclo, more preferably phenyl. Alternatively, while R₁₀ₐ and X₃ are selected from among these, in one embodiment X₅ is selected from -COX₁₀ wherein X₁₀ is phenyl, alkyl or heterocyclo, more preferably phenyl, or X₅ is -COOX₁₀ wherein X₁₀ is alkyl, preferably t-butyl. Among the more preferred embodiments, therefore, are taxanes corresponding to structure 2 in which (i) X₅ is -COOX₁₀ wherein X₁₀ is tert-butyl or X₅ is -COX₁₀ wherein X₁₀ is phenyl, (ii) X₃ is substituted or unsubstituted heterocyclo, more preferably substituted or unsubstituted, furyl, thienyl, or pyridyl, still more preferably unsubstituted, furyl, thienyl or pyridyl, and (iii) R₇ₐ is unsubstituted ethyl or propyl, more preferably ethyl.

Among the preferred embodiments, therefore, are taxanes corresponding to structure 1 or 2 wherein R₁₀ is R₁₀ₐCOO- wherein R₁₀ₐ is ethyl. In this embodiment, X₃ is preferably heterocyclo, still more preferably furyl, thienyl or pyridyl; and X₅ is preferably benzoyl, alkoxycarbonyl, or heterocyclocarbonyl, more preferably benzoyl, t-butoxycarbonyl or t-amyloxycarbonyl, In one alternative of this embodiment, X₃ is heterocyclo; X₅ is benzoyl, alkoxycarbonyl, or heterocyclocarbonyl, more preferably benzoyl, t-butoxycarbonyl or t-amyloxycarbonyl, still more preferably t-butoxycarbonyl; R₂ is benzoyl, *R₉ is keto and R₁₄ is hydrogen. In another alternative of this embodiment, X₃ is heterocyclo; X₅ is benzoyl, alkoxycarbonyl, or heterocyclocarbonyl, more preferably benzoyl, t-butoxycarbonyl or τ-amyloxycarbonyl, still more preferably t-butoxycarbonyl; R₂ is benzoyl, R₉ is keto and R₁₄ is hydrogen. In another alternative of this embodiment, X₃ is heterocyclo; X₅ is benzoyl, alkoxycarbonyl, or heterocyclocarbonyl, more preferably benzoyl, t-butoxycarbonyl or t-amyloxycarbonyl, still more preferably t-butoxycarbonyl; R₂ is benzoyl, R₉ is keto and R₁₄ is hydroxy. In another alternative of this embodiment, X₃ is heterocyclo; X₅ is benzoyl, alkoxycarbonyl, or heterocyclocarbonyl, more preferably benzoyl, t-butoxycarbonyl or t-amyloxycarbonyl, still more preferably t-butoxycarbonyl; R₂ is benzoyl, R₉ is hydroxy and R₁₄ is hydroxy. In another alternative of this embodiment, X₃ is heterocyclo; X₅ is benzoyl, alkoxycarbonyl, or heterocyclocarbonyl, more preferably benzoyl, t-butoxycarbonyl or t-amyloxycarbonyl, still more preferably t-butoxycarbonyl); R₂ is benzoyl, R₉ is hydroxy and R₁₄ is hydrogen. In another alternative of this embodiment, X₃ is heterocyclo; X₅ is benzoyl, alkoxycarbonyl, or heterocyclocarbonyl, more preferably benzoyl, t-butoxycarbonyl or t-amyloxycarbonyl, still more preferably t-butoxycarbonyl; R₂ is benzoyl, R₉ is acyloxy and R₁₄ is hydroxy, In another alternative of this embodiment, X₃ is heterocyclo; X₅ is benzoyl, alkoxycarbonyl, or heterocyclocarbonyl, more preferably benzoyl, t-butoxycarbonyl or t-amyloxycarbonyl, still more preferably t-butoxycarbonyl; R₂ is benzoyl, R₉ is acyloxy and R₁₄ is hydrogen. In each of the alternatives of this embodiment when the taxane has structure 1, R₇ and R₁₀ may each have the beta stereochemical configuration, R₇ and R₁₀ may each have the alpha stereochemical configuration, R₇ may have the alpha stereochemical configuration while R₁₀ has the beta stereochemical configuration or R₇ may have the beta stereochemical configuration while R₁₀ has the alpha stereochemical configuration.

Also among the preferred embodiments are taxanes corresponding to structure 1 or 2 wherein R₁₀ is R₁₀ₐCOO- wherein R₁₀ₐ is propyl. In this embodiment, X₃ is preferably heterocyclo, still more preferably furyl, thienyl or pyridyl; and X₅ is preferably benzoyl, alkoxycarbonyl, or heterocyclocarbonyl, more preferably benzoyl, t-butoxycarbonyl or t-amyloxycarbonyl. In one alternative of this embodiment, X₃ is heterocyclo; X₅ is benzoyl, alkoxycarbonyl, or heterocyclocarbonyl, more preferably benzoyl, t-butoxycarbonyl or t-amyloxycarbonyl, still more preferably t-butoxycarbonyl; R₂ is benzyl, R₉ is keto and R₁₄ is hydrogen. In another alternative of this embodiment_{;} X₃ is heterocyclo; X₅ is benzoyl, alkoxycarbonyl; or heterocyclocarbonyl, more preferably benzoyl, t-butoxycarbonyl or t-amyloxycarbonyl, still more preferably t-butoxycarbonyl; R₂ is benzoyl, R₉ is keto and R₁₄ is hydrogen. In another alternative of this embodiment, X₃ is heterocyclo; X₅ is benzoyl, alkoxycarbonyl, or heterocyclocarbonyl, more preferably benzoyl, t-butoxycarbonyl or t-amyloxycarbonyl, still more preferably t-butoxycarbonyl; R₂ is benzoyl, R₉ is keto and R₁₄ is hydroxy. In another alternative of this embodiment, X₃ is heterocyclo; X₅ is benzoyl, alkoxycarbonyl, or heterocyclocarbonyl, more preferably benzoyl, t-butoxycarbonyl or t-amyloxycarbonyl, still more preferably t-butoxycarbonyl; R₂ is benzoyl, R₉ is hydroxy and R₁₄ is hydroxy. In another alternative of this embodiment, X₃ is heterocyclo; X₅ is benzoyl, alkoxycarbonyl, or heterocyclocarbonyl, more preferably benzoyl, t-butoxycarbonyl or t-amyloxycarbonyl, still more preferably t-butoxycarbonyl; R₂ is benzoyl, R₉ is hydroxy and R₁₄ is hydrogen. In another alternative of this embodiment, X₃ is heterocyclo; X₅ is benzoyl, alkoxycarbonyl, or heterocyclocarbonyl, more preferably benzoyl, t-butoxycarbonyl or t-amyloxycarbonyl, still more preferably t-butoxycarbonyl; R₂ is benzoyl, R₉ is acyloxy and R₁₄ is hydroxy. In another alternative of this embodiment, X₃ is heterocyclo; X₅ is benzoyl, alkoxycarbonyl, or heterocyclocarbonyl, more preferably benzoyl, t-butoxycarbonyl or t-amyloxycarbonyl, still more preferably t-butoxycarbonyl; R₂ is benzoyl, R₉ is acyloxy and R₁₄ is hydrogen. In each of the alternatives of this embodiment when the taxane has structure 1, R₇ and R₁₀ may each have the beta stereochemical configuration, R₇ and R₁₀ may each have the alpha stereochemical configuration, R₇ may have the alpha stereochemical configuration while R₁₀ has the beta stereochemical configuration or R₇ may have the beta stereochemical configuration while R₁₀ has the alpha stereochemical configuration.

Taxanes having the general formula 1 may be obtained by treatment of a β-lactam with an alkoxide having the taxane tetracyclic nucleus and a C-13 metallic oxide substituent to form compounds having a β-amido ester substituent at C-13 (as described more fully in Holton U.S. Patent 5,466,834), followed by removal of the hydroxy protecting groups. The β-lactam has the following structural formula (3): 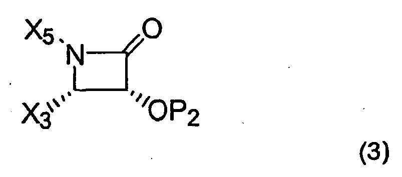 wherein P₂ is a hydroxy protecting group and X₃ and X₅ are as previously defined and the alkoxide has the structural formula (4): 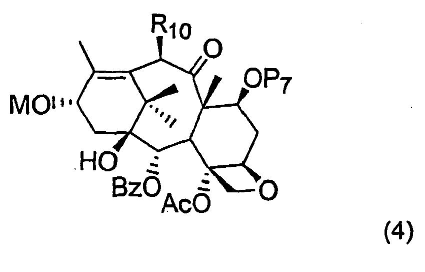 wherein M is a metal or ammonium, P₇ is a hydroxy protecting group and R₁₀ is as previously defined.

Alkoxide 4 may be prepared from 10-deacetylbaccatin III (or a derivative thereof) by selective protection of the C(7) hydroxyl group and then esterification of the C(10) hydroxyl group followed by treatment with a metallic amide. In one embodiment of the present invention, the C(7) hydroxyl group of 10-deacetylbaccatin III is selectively protected with a silyl group as described, for example, by Denis, et. al. (J. Am. Chem. Soc., 1988, 110, 5917). In general, the silylating agents may be used either alone or in combination with a catalytic amount of a base such as an alkali metal base.

Alternatively, the C(10) hydroxyl group of a taxane can be selectively acylated in the absence of a base, as described, for example in Holton et al., PCT Patent Application WO 99/09021. Acylating agents which may be used for the selective acylation of the C(10) hydroxyl group of a taxane include substituted or unsubstituted alkyl or aryl anhydrides. While the acylation of the C(10) hydroxy group of the taxane will proceed at an adequate rate for many acylating agents, it has been discovered that the reaction rate may be increased by including a Lewis acid in the reaction mixture. Preferred Lewis acids include zinc chloride, stannic chloride, cerium trichloride, cuprous chloride, lanthanum trichloride, dysprosium trichloride, and ytterbium trichloride. Zinc chloride or cerium trichloride is particularly preferred when the acylating agent is an anhydride.

Derivatives of 10-deacetylbaccatin III having alternative substituents at C(2), C(9) and C(14) and processes for their preparation are known in the art. Taxane derivatives having acyloxy substituents other than benzoyloxy at C(2) may be prepared, for example, as described in Holton et al., U.S. Patent No. 5,728,725 or Kingston et al., U.S. Patent No. 6,002,023. Taxanes having acyloxy or hydroxy substituents at C(9) in place of keto may be prepared, for example as described in Holton et al., U.S. Patent No. 6,011,056 or Gunawardana et al., U.S. Patent No. 5,352,806. Taxanes having a beta hydroxy substituent at C(14) may be prepared from naturally occurring 14-hydroxy-10-deacetylbaccatin III.

Processes for the preparation and resolution of the β-lactam starting material are generally well known. For example, the β-lactam may be prepared as described in Holton, U.S. Patent No. 5,430,160 and the resulting enatiomeric mixtures of β-lactams may be resolved by a stereoselective hydrolysis using a lipase or enzyme as described, for example, in Patel, U.S. Patent No. 5,879,929 Patel U.S. Patent No. 5,567,614 or a liver homogenate as described, for example, in PCT Patent Application No. 00l41204. In a preferred embodiment in which the β-lactam is furyl substituted at the C(4) position, the β-lactam can be prepared as illustrated in the following reaction scheme: 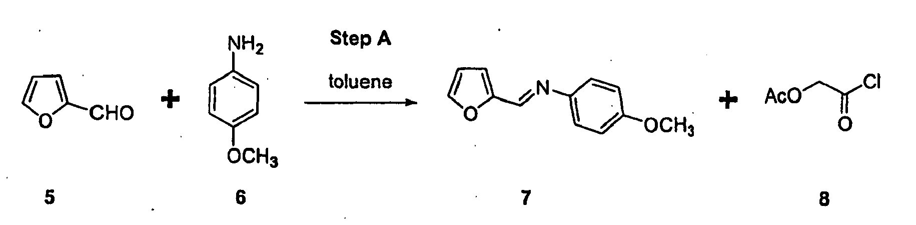 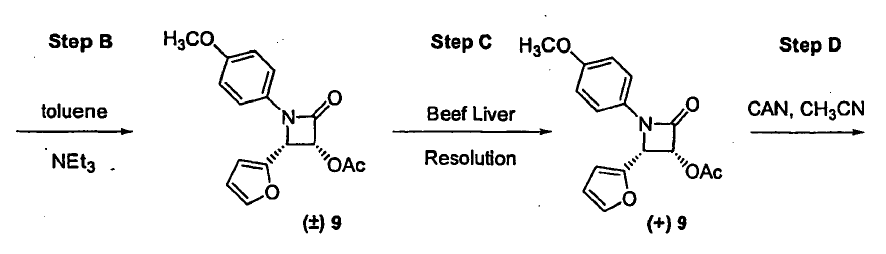 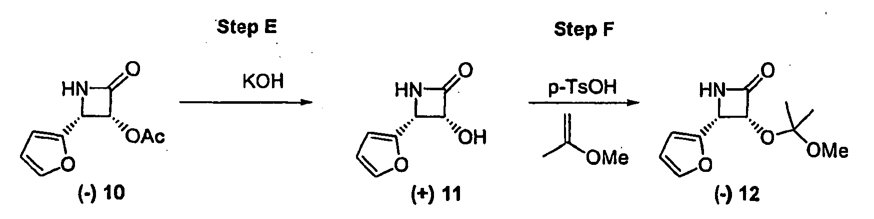 wherein Ac is acetyl, NEt₃ is triethylamine, CAN is ceric ammonium nitrate, and p-TsOH is p-toluenesulfonic acid. The beef liver resolution may be carried out, for example, by combining the enatiomeric β-lactam mixture with a beef liver suspension (prepared, for example, by adding 20 g of frozen beef liver to a blender and then adding a pH 8 buffer to make a total volume of 1 L).

Compounds of formula 1 of the instant invention are useful for inhibiting tumor growth in mammals including humans and are preferably administered in the form of a pharmaceutical composition comprising an effective antitumor amount of a compound of the instant invention in combination with at least one pharmaceutically or pharmacologically acceptable carrier. The carrier, also known in the art as an excipient, vehicle, auxiliary, adjuvant, or diluent, is any substance which is pharmaceutically inert, confers a suitable consistency or form to the composition, and does not diminish the therapeutic efficacy of the antitumor compounds. The carrier is "pharmaceutically or pharmacologically acceptable" if it does not produce an adverse, allergic or other untoward reaction when administered to a mammal or human, as appropriate.

The pharmaceutical compositions containing the antitumor compounds of the present invention may be formulated in any conventional manner. Proper formulation is dependent upon the route of administration chosen. The compositions of the invention can be formulated for any route of administration so long as the target tissue is available via that route. Suitable routes of administration include, but are not limited to, oral, parenteral (e.g., intravenous, intraarterial, subcutaneous, rectal, subcutaneous, intramuscular, intraorbital, intracapsular, intraspinal, intraperitoneal, or intrastemal), topical (nasal, transdermal, intraocular), intravesical, intrathecal, enteral, pulmonary, intralymphatic, intracavital, vaginal, transurethral, intradermal, aural, intramammary, buccal, orthotopic, intratracheal, intralesional, percutaneous, endoscopical, transmucosal, sublingual and intestinal administration.

Pharmaceutically acceptable carriers for use in the compositions of the present invention are well known to those of ordinary skill in the art and are selected based upon a number of factors: the particular antitumor compound used, and its concentration, stability and intended bioavailability; the disease, disorder or condition being treated with the composition; the subject, its age, size and general condition; and the route of administration. Suitable carriers are readily determined by one of ordinary skill in the art (see, for example, J. G. Nairn, in: Remington's Pharmaceutical Science (A. Gennaro, ed.), Mack Publishing Co., Easton, Pa., (1985), pp. 1492-1517, the contents of which are incorporated herein by reference).

The compositions are preferably formulated as tablets, dispersible powders, pills, capsules, gelcaps, caplets, gels, liposomes, granules, solutions, suspensions, emulsions, syrups, elixirs, troches, dragees, lozenges, or any other dosage form which can be administered orally. Techniques and compositions for making oral dosage forms useful in the present invention are described in the following references: 7 Modern Pharmaceutics, Chapters 9 and 10 (Banker & Rhodes, Editors, 1979); Lieberman et al., Pharmaceutical Dosage Forms: Tablets (1981); and Ansel, Introduction to Pharmaceutical Dosage Forms 2nd Edition (1976).

The compositions of the invention for oral administration comprise an effective antitumor amount of a compound of the invention in a pharmaceutically acceptable carrier. Suitable carriers for solid dosage forms include sugars, starches, and other conventional substances including lactose, talc; sucrose, gelatin, carboxymethylcellulose, agar, mannitol, sorbitol, calcium phosphate, calcium carbonate, sodium carbonate, kaolin, alginic acid, acacia, corn starch, potato starch, sodium saccharin, magnesium carbonate, tragacanth, microcrystalline cellulose, colloidal silicon dioxide, croscarmellose sodium, talc, magnesium stearate, and stearic acid. Further, such solid dosage forms may be uncoated or may be coated by known techniques; e.g., to delay disintegration and absorption.

The antitumor compounds of the present invention are also preferably formulated for parenteral administration, e.g., formulated for injection via intravenous, intraarterial, subcutaneous, rectal, subcutaneous, intramuscular, intraorbital, intracapsular, intraspinal, intraperitoneal, or intrasternal routes. The compositions of the invention for parenteral administration comprise an effective antitumor amount of the antitumor compound in a pharmaceutically acceptable carrier. Dosage forms suitable for parenteral administration include solutions, suspensions, dispersions, emulsions or any other dosage form which can be administered parenterally. Techniques and compositions for making parenteral dosage forms are known in the art.

Suitable carriers used in formulating liquid dosage forms for oral or parenteral administration include nonaqueous, pharmaceutically-acceptable polar solvents such as oils, alcohols, amides, esters, ethers, ketones, hydrocarbons and mixtures thereof, as well as water, saline solutions, dextrose solutions (e.g., DW5), electrolyte solutions, or any other aqueous, pharmaceutically acceptable liquid.

Suitable nonaqueous, pharmaceutically-acceptable polar solvents include, but are not limited to, alcohols (e.g., α-glycerol formal, β-glycerol formal, 1, 3-butyleneglycol, aliphatic or aromatic alcohols having 2-30 carbon atoms such as methanol, ethanol, propanol, isopropanol, butanol, t-butanol, hexanol, octanol, amylene hydrate, benzyl alcohol, glycerin (glycerol), glycol, hexylene glycol, tetrahydrofurfuryl alcohol, lauryl alcohol, cetyl alcohol, or stearyl alcohol, fatty acid esters of fatty alcohols such as polyalkylene glycols (e.g., polypropylene glycol, polyethylene glycol), sorbitan, sucrose and cholesterol); amides (e.g., dimethylacetamide (DMA), benzyl benzoate DMA, dimethylformamide, N-(β-hydroxyethyl)-)actamide, N,N-dimethylacetamide amides, 2-pyrrolidinone, 1-methyl-2-pyrrolidinone, or polyvinylpyrrolidone); esters (e.g., 1-methyl-2-pyrrolidinone, 2-pyrrolidinone, acetate esters such as monoacetin, diacetin, and triacetin, aliphatic or aromatic esters such as ethyl caprylate or octanoate, alkyl oleate, benzyl benzoate, benzyl acetate, dimethylsulfoxide (DMSO), esters of glycerin such as mono, di, or tri-glyceryl citrates or tartrates, ethyl benzoate, ethyl acetate, ethyl carbonate, ethyl lactate, ethyl oleate, fatty acid esters of sorbitan, fatty acid derived PEG esters, glyceryl monostearate, glyceride esters such as mono, di, or tri-glycerides, fatty acid esters such as isopropyl myristrate, fatty acid derived PEG esters such as PEG-hydroxyoleate and PEG-hydroxystearate, N-methyl pyrrolidinone, pluronic 60, polyoxyethylene sorbitol oleic polyesters such as poly(ethoxylated)₃₀₋₆₀ sorbitol poly(oleate)_{2-4.} poly(oxyethylene)₁₅₋₂₀ monooleate, poly(oxyethylene)₁₅₋₂₀ mono 12-hydroxystearate, and poly(oxyethylene)₁₅₋₂₀ mono ricinoleate, polyoxyethylene sorbitan esters such as polyoxyethylene-sorbitan monooleate, polyoxyethylene-sorbitan monopalmitate, polyoxyethylene-sorbitan monolaurate, polyoxyethylene-sorbitan monostearate, and Polysorbate® 20, 40, 60 or 80 from ICI Americas, Wilmington, DE, polyvinylpyrrolidone, alkyleneoxy modified fatty acid esters such as polyoxyl 40 hydrogenated castor oil and polyoxyethylated castor oils (e.g., Cremophor® EL solution or Cremophor® RH 40 solution), saccharide fatty acid esters (i.e., the condensation product of a monosaccharide (e.g., pentoses such as ribose, ribulose, arabinose, xylose, lyxose and xylulose, hexoses such as glucose, fructose, galactose, mannose and sorbose, trioses, tetroses, heptoses, and octoses), disaccharide (e.g., sucrose, maltose, lactose and trehalose) or oligosaccharide or mixture thereof with a C₄ C22 fatty acid(s)(e.g., saturated fatty acids such as caprylic acid, capric acid, lauric acid, myristic acid, palmitic acid and stearic acid, and unsaturated fatty acids such as palmitoleic acid, oleic acid, elaidic acid, erucic acid and linoleic acid)), or steroidal esters); alkyl, aryl, or cyclic ethers having 2-30 carbon atoms (e.g., diethyl ether, tetrahydrofuran, dimethyl isosorbide, diethylene glycol monoethyl ether); glycofurol (tetrahydrofurfuryl alcohol polyethylene glycol ether); ketones having 3-30 carbon atoms (e.g., acetone, methyl ethyl ketone, methyl isobutyl ketone); aliphatic, cycloaliphatic or aromatic hydrocarbons having 4-30 carbon atoms (e.g., benzene, cyclohexane, dichloromethane, dioxolanes, hexane, n-decane, n-dodecane, n-hexane, sulfolane, tetramethylenesulfon, tetramethylenesulfoxide, toluene, dimethylsulfoxide (DMSO), or tetramethylenesulfoxide); oils of mineral, vegetable, animal, essential or synthetic origin (e.g., mineral oils such as aliphatic or wax-based hydrocarbons, aromatic hydrocarbons, mixed aliphatic and aromatic based hydrocarbons, and refined paraffin oil, vegetable oils such as linseed, tung, safflower, soybean, castor, cottonseed, groundnut, rapeseed, coconut, palm, olive, corn, corn germ, sesame, persic and peanut oil and glycerides such as mono-, di- or triglycerides, animal oils such as fish, marine, sperm, cod-liver, haliver, squalene, squalane, and shark liver oil, oleic oils, and polyoxyethylated castor oil); alkyl or aryl halides having 1-30 carbon atoms and optionally more than one halogen substituent; methylene chloride; monoethanolamine; petroleum benzin; trolamine; omega-3 polyunsaturated fatty acids (e.g., alpha-linolenic acid, eicosapentaenoic acid, docosapentaenoic acid, or docosahexaenoic acid); polyglycol ester of 12-hydroxystearic acid and polyethylene glycol (Solutol® HS-15, from BASF, Ludwigshafen, Germany); polyoxyethylene glycerol; sodium laurate; sodium oleate; or sorbitan monooleate.

Other pharmaceutically acceptable solvents for use in the invention are well known to those of ordinary skill in the art, and are identified in The Chemotherapy Source Book (Williams & Wilkens Publishing), The Handbook of Pharmaceutical Excipients, (American Pharmaceutical Association, Washington, D.C., and The Pharmaceutical Society of Great Britain, London, England; 1968), Modern Pharmaceutics, (G. Banker et al., eds., 3d ed.)(Marcel Dekker, Inc., New York; New York, 1995), The Pharmacological Basis of Therapeutics, (Goodman & Gilman, McGraw Hill Publishing), Pharmaceutical Dosage Forms, (H. Lieberman et al., eds., )(Marcel Dekker, Inc., New York, New York, 1980), Remington's Pharmaceutical Sciences (A. Gennaro, ed:, 19th ed.)(Mack Publishing, Easton, PA, 1995), The United States Pharmacopeia 24, The National Formulary 19, (National Publishing, Philadelphia, PA, 2000), A.J. Spiegel et al., and Use of Nonaqueous Solvents in Parenteral Products, JOURNAL OF PHARMACEUTICAL SCIENCES, Vol. 52, No. 10, pp. 917-927 (1963).

Preferred solvents include those known to stabilize the antitumor compounds, such as oils rich in triglycerides, for example, safflower oil, soybean oil or mixtures thereof, and alkyleneoxy modified fatty acid esters such as polyoxyl 40 hydrogenated castor oil and polyoxyethylated castor oils (e.g., Cremophor® EL solution or Cremophor® RH 40 solution). Commercially available triglycerides include Intralipid® emulsified soybean oil (Kabi-Pharmacia Inc., Stockholm, Sweden), Nutralipid ® emulsion (McGaw, Irvine, California), Liposyn® II 20% emulsion (a 20% fat emulsion solution containing 100 mg safflower oil, 100 mg soybean oil, 12 mg egg phosphatides, and 25 mg glycerin per ml of solution; Abbott Laboratories, Chicago, Illinois), Liposyn® III 2% emulsion (a 2% fat emulsion solution containing 100 mg safflower oil, 100 mg soybean oil, 12 mg egg phosphatides, and 25 mg glycerin per ml of solution; Abbott Laboratories, Chicago, Illinois), natural or synthetic glycerol derivatives containing the docosahexaenoyl group at levels between 25% and 100% by weight based on the total fatty acid content (Dhasco® (from Martek Biosciences Corp., Columbia, MD), DHA Maguro® (from Daito Enterprises, Los Angeles, CA), Soyacal®, and Travemulsion®. Ethanol is a preferred solvent for use in dissolving the antitumor compound to form solutions, emulsions, and the like.

Additional minor components can be included in the compositions of the invention for a variety of purposes well known in the pharmaceutical industry. These components will for the most part impart properties which enhance retention of the antitumor compound at the site of administration, protect the stability of the composition, control the pH, facilitate processing of the antitumor compound into pharmaceutical formulations, and the like. Preferably, each of these components is individually present in less than about 15 weight % of the total composition, more preferably less than about 5 weight %, and most preferably less than about 0.5 weight % of the total composition. Some components, such as fillers or diluents, can constitute up to 90 wt.% of the total composition, as is well known in the formulation art. Such additives include cryoprotective agents for preventing reprecipitation of the taxane, surface active, wetting or emulsifying agents (e.g., lecithin, polysorbate-80, Tween® 80, pluronic 60, polyoxyethylene stearate ), preservatives (e.g., ethyl-p-hydroxybenzoate), microbial preservatives (e.g., benzyl alcohol, phenol, m-cresol, chlorobutanol, sorbic acid, thimerosal and paraben), agents for adjusting pH or buffering agents (e.g., acids, bases, sodium acetate, sorbitan monolaurate), agents for adjusting osmolarity (e.g., glycerin), thickeners (e.g., aluminum monostearate, stearic acid, cetyl alcohol, stearyl alcohol, guar gum, methyl cellulose, hydroxypropylcellulose, tristearin, cetyl wax esters, polyethylene glycol), colorants, dyes, flow aids, non-volatile silicones (e.g., cyclomethicone), clays (e.g., bentonites), adhesives, bulking agents, flavorings, sweeteners, adsorbents, fillers (e.g., sugars such as lactose, sucrose, mannitol, or sorbitol, cellulose, or calcium phosphate), diluents (e.g., water, saline, electrolyte solutions), binders (e.g., starches such as maize starch, wheat starch, rice starch, or potato starch, gelatin, gum tragacanth, methyl cellulose, hydroxypropyl methylcellulose, sodium carboxymethyl cellulose, polyvinylpyrrolidone, sugars, polymers, acacia), disintegrating agents (e.g., starches such as maize starch, wheat starch, rice starch, potato starch, or carboxymethyl starch, cross-linked polyvinyl pyrrolidone, agar, alginic acid or a salt thereof such as sodium alginate, croscarmellose sodium or crospovidone), lubricants (e.g., silica, talc, stearic acid or salts thereof such as magnesium stearate, or polyethylene glycol), coating agents (e.g., concentrated sugar solutions including gum arabic, talc, polyvinyl pyrrolidone, carbopol gel, polyethylene glycol, or titanium dioxide), and antioxidants (e.g., sodium metabisulfite, sodium bisulfite, sodium sulfite, dextrose, phenols, and thiophenols).

In a preferred embodiment, a pharmaceutical composition of the invention comprises at least one nonaqueous, pharmaceutically acceptable solvent and an antitumor compound having a solubility in ethanol of at least about 100, 200, 300, 400, 500, 600, 700 or 800 mg/ml. While not being bound to a particular theory, it is believed that the ethanol solubility of the antitumor compound may be directly related to its efficacy. The antitumor compound can also be capable of being crystallized from a solution. In other words, a crystalline antitumor compound, such as compound 1393, can be dissolved in a solvent to form a solution and then recrystallized upon evaporation of the solvent without the formation of any amorphous antitumor compound. It is also preferred that the antitumor compound have an ID50 value (i.e, the drug concentration producing 50% inhibition of colony formation) of at least 4, 5, 6, 7, 8, 9, or 10 times less that of paclitaxel when measured according to the protocol set forth in the working examples.

Dosage form administration by these routes may be continuous or intermittent, depending, for example, upon the patient's physiological condition, whether the purpose of the administration is therapeutic or prophylactic, and other factors known to and assessable by a skilled practitioner.

Dosage and regimens for the administration of the pharmaceutical compositions of the invention can be readily determined by those with ordinary skill in treating cancer. It is understood that the dosage of the antitumor compounds will be dependent upon the age, sex, health, and weight of the recipient, kind of concurrent treatment, if any, frequency of treatment, and the nature of the effect desired. For any mode of administration, the actual amount of antitumor compound delivered, as well as the dosing schedule necessary to achieve the advantageous effects described herein, will also depend, in part, on such factors as the bioavailability of the antitumor compound, the disorder being treated, the desired therapeutic dose, and other factors that will be apparent to those of skill in the art. The dose administered to an animal, particularly a human, in the context of the present invention should be sufficient to effect the desired therapeutic response in the animal over a reasonable period of time. Preferably, an effective amount of the antitumor compound, whether administered orally or by another route, is any amount which would result in a desired therapeutic response when administered by that route. Preferably, the compositions for oral administration are prepared in such a way that a single dose in one or more oral preparations contains at least 20 mg of the antitumor compound per m² of patient body surface area, or at least 50,100,150, 200, 300; 400, or 500 mg of the antitumor compound per m² of patient body surface area, wherein the average body surface area for a human is 1.8 m². Preferably, a single dose of a composition for oral administration contains from about 20 to about 600 mg of the antitumor compound per m² of patient body surface area, more preferably from about 25 to about 400 mg/m². even more preferably, from about 40 to about 300 mg/m², and even more preferably from about 50 to about 200 mg/m². Preferably, the compositions for parenteral administration are prepared in such a way that a single dose contains at least 20 mg of the antitumor compound per m² of patient body surface area, or at least 40, 50, 100, 150, 200, 300, 400, or 500 mg of the antitumor compound per m² of patient body surface area. Preferably, a single dose in one or more parenteral preparations contains from about 20 to about 500 mg of the antitumor compound per m² of patient body surface area, more preferably from about 40 to about 400 mg/m². and even more preferably, from about 60 to about 350 mg/m². However, the dosage may vary depending on the dosing schedule which can be adjusted as necessary to achieve the desired therapeutic effect. It should be noted that the ranges of effective doses provided herein are not intended to limit the invention and represent preferred dose ranges. The most preferred dosage will be tailored to the individual subject, as is understood and determinable by one of ordinary skill in the art without undue experimentation.

The concentration of the antitumor compound in a liquid pharmaceutical composition is preferably between about 0.01 mg and about 10 mg per ml of the composition, more preferably between about 0.1 mg and about 7 mg per ml, even more preferably between about 0.5 mg and about 5 mg per ml, and most preferably between about 1.5 mg and about 4 mg per ml. Relatively low concentrations are generally preferred because the antitumor compound is most soluble in the solution at low concentrations. The concentration of the antitumor compound in a solid pharmaceutical composition for oral administration is preferably between about 5 weight % and about 50 weight %, based on the total weight of the composition, more preferably between about 8 weight % and about 40 weight %, and most preferably between about 10 weight % and about 30 weight %.

In one embodiment, solutions for oral administration are prepared by dissolving an antitumor compound in any pharmaceutically acceptable solvent capable of dissolving the compound (e.g., ethanol or methylene chloride) to form a solution. An appropriate volume of a carrier which is a solution, such as Cremophor® EL solution, is added to the solution while stirring to form a pharmaceutically acceptable solution for oral administration to a patient. If desired: such solutions can be formulated to contain a minimal amount of, or to be free of, ethanol, which is known in the art to cause adverse physiological effects when administered at certain concentrations in oral formulations.

In another embodiment, powders or tablets for oral administration are prepared by dissolving an antitumor compound in any pharmaceutically acceptable solvent capable of dissolving the compound (e.g.,ethahol or methylene chloride) to form a solution. The solvent can optionally be capable of evaporating when the solution is dried under vacuum. An additional carrier can be added to the solution prior to drying, such as Cremophor® EL solution. The resulting solution is dried under vacuum to form a glass. The glass is then mixed with a binder to form a powder. The powder can be mixed with fillers or other conventional tabletting agents and processed to form a tablet for oral administration to a patient. The powder can also be added to any liquid carrier as described above to form a solution, emulsion, suspension or the like for oral administration.

Emulsions for parenteral administration can be prepared by dissolving an antitumor compound in any pharmaceutically acceptable solvent capable of dissolving the compound (e.g., ethanol or methylene chloride) to form a solution. An appropriate volume of a carrier which is an emulsion, such as Liposyn® II or Liposyn® III emulsion, is added to the solution while stirring to form a pharmaceutically acceptable emulsion for parenteral administration to a patient. If desired, such emulsions can be formulated to contain a minimal amount of, or to be free of, ethanol or Cremophor® solution, which are known in the art to cause adverse physiological effects when administered at certain concentrations in parenteral formulations.

Solutions for parenteral administration can be prepared by dissolving an antitumor compound in any pharmaceutically acceptable solvent capable of dissolving the compound (e.g., ethanol or methylene chloride) to form a solution. An appropriate volume of a carrier which is a solution, such as Cremophor® solution, is added to the solution while stirring to form a pharmaceutically acceptable solution for parenteral administration to a patient. If desired, such solutions can be formulated to contain a minimal amount of, or to be free of, ethanol or Cremophor® solution, which are known in the art to cause adverse physiological effects when administered at certain concentrations in parenteral formulations.

If desired, the emulsions or solutions described above for oral or parenteral administration can be packaged in IV bags, vials or other conventional containers in concentrated form and diluted with any pharmaceutically acceptable liquid, such as saline, to form an acceptable taxane concentration prior to use as is known in the art.

### Definitions

The terms "hydrocarbon" and "hydrocarbyl" as used herein describe organic compounds or radicals consisting exclusively of the elements carbon and hydrogen. These moieties include alkyl, alkenyl, alkynyl, and aryl moieties. These moieties also include alkyl, alkenyl, alkynyl, and aryl moieties substituted with other aliphatic or cyclic hydrocarbon groups, such as alkaryl, alkenaryl and alkynaryl. Unless otherwise indicated, these moieties preferably comprise 1 to 20 carbon atoms.

The "substituted hydrocarbyl" moieties described herein are hydrocarbyl moieties which are substituted with at least one atom other than carbon, including moieties in which a carbon chain atom is substituted with a hetero atom such as nitrogen, oxygen, silicon, phosphorous, boron, sulfur, or a halogen atom. These substituents include halogen, heterocyclo, alkoxy, alkenoxy, alkynoxy, aryloxy, hydroxy, protected hydroxy, keto, acyl, acyloxy, nitro, amino, amido, nitro, cyano, thiol, ketals, acetals, esters and ethers.

The term "heteroatom" shall mean atoms other than carbon and hydrogen.

The "heterosubstituted methyl" moieties described herein are methyl groups in which the carbon atom is covalently bonded to at least one heteroatom and optionally with hydrogen, the heteroatom being, for example, a nitrogen, oxygen, silicon, phosphorous, boron, sulfur, or halogen atom. The heteroatom may, in turn, be substituted with other atoms to form a heterocyclo, alkoxy, alkenoxy, alkynoxy, aryloxy, hydroxy, protected hydroxy, oxy, acyloxy, nitro, amino, amido, thiol, ketals, acetals, esters or ether moiety.

The "heterosubstituted acetate" moieties described herein are acetate groups in which the carbon of the methyl group is covalently bonded to at least one heteroatom and optionally with hydrogen, the heteroatom being, for example, as nitrogen, oxygen, silicon, phosphorous, boron, sulfur, or halogen atom. The heteroatom may, in turn, be substituted with other atoms to form a heterocyclo, alkoxy, alkenoxy, alkynoxy, aryloxy, hydroxy, protected hydroxy, oxy, acyloxy, nitro, amino, amido, thiol, ketals, acetals, esters or ether moiety.

Unless otherwise indicated, the alkyl groups described herein are preferably lower alkyl containing from one to eight carbon atoms in the principal chain and up to 20 carbon atoms. They may be straight or branched chain or cyclic and include methyl, ethyl, propyl, isopropyl, butyl, hexyl and the like.

Unless otherwise indicated, the alkenyl groups described herein are preferably lower alkenyl containing from two to eight carbon atoms in the principal chain and up to 20 carbon atoms. They may be straight or branched chain or cyclic and include ethenyl, propenyl, isopropenyl, butenyl, isobutenyl, hexenyl, and the like.

Unless otherwise indicated, the alkynyl groups described herein are preferably lower alkynyl containing from two to eight carbon atoms in the principal chain and up to 20 carbon atoms. They may be straight or branched chain and include ethynyl, propynyl, butynyl, isobutynyl, hexynyl, and the like.

The terms "aryl" or "ar" as used herein alone or as part of another group denote optionally substituted homocyclic aromatic groups, preferably monocyclic or bicyclic groups containing from 6 to 12 carbons in the ring portion, such as phenyl, biphenyl, naphthyl, substituted phenyl, substituted biphenyl or substituted naphthyl. Phenyl and substituted phenyl are the more preferred aryl.

The terms "halogen" or "halo" as used herein alone or as part of another group refer to chlorine, bromine, fluorine, and iodine.

The terms "heterocyclo" or "heterocyclic" as used herein alone or as part of another group denote optionally substituted, fully saturated or unsaturated, monocyclic or bicyclic, aromatic or nonaromatic groups having at least one heteroatom in at least one ring, and preferably 5 or 6 atoms in each ring. The heterocyclo group preferably has 1 or 2 oxygen atoms, 1 or 2 sulfur atoms, and/or 1 to 4 nitrogen atoms in the ring, and may be bonded to the remainder of the molecule through a carbon or heteroatom. Exemplary heterocyclo include heteroaromatics such as furyl, thienyl, pyridyl, oxazolyl, pyrrolyl, indolyl, quinolinyl, or isoquinolinyl and the like. Exemplary substituents include one or more of the following groups: hydrocarbyl, substituted hydrocarbyl, keto, hydroxy, protected hydroxy, acyl, acyloxy, alkoxy, alkenoxy, alkynoxy, aryloxy, halogen, amido, amino, nitro, cyano, thiol, ketals, acetals, esters and ethers.

The term "heteroaromatic" as used herein alone or as part of another group denote optionally substituted aromatic groups having at least one heteroatom in at least one ring, and preferably 5 or 6 atoms in each ring. The heteroaromatic group preferably has 1 or 2 oxygen atoms, 1 or 2 sulfur atoms, and/or 1 to 4 nitrogen atoms in the ring, and may be bonded to the remainder of the molecule through a carbon or heteroatom. Exemplary heteroaromatics include furyl, thienyl, pyridyl, oxazolyl, pyrrolyl, indolyl, quinolinyl, or isoquinolinyl and the like. Exemplary substituents include one or more of the following groups: hydrocarbyl, substituted hydrocarbyl, keto, hydroxy, protected hydroxy, acyl, acyloxy, alkoxy, alkenoxy, alkynoxy, aryloxy, halogen, amido, amino, nitro, cyano, thiol, ketals, acetals, esters and ethers.

The term "acyl," as used herein alone or as part of another group, denotes the moiety formed by removal of the hydroxyl group from the group -COOH of an organic carboxylic acid, e.g., RC(O)-, wherein R is R¹, R¹O-, R¹R²N-, or R¹S-, R¹ is hydrocarbyl, heterosubstituted hydrocarbyl, or heterocyclo and R² is hydrogen, hydrocarbyl or substituted hydrocarbyl.

The term "acyloxy," as used herein alone or as part of another group, denotes an acyl group as described above bonded through an oxygen linkage (-O-), e.g., RC(O)O- wherein R is as defined in connection with the term "acyl."

Unless otherwise indicated, the alkoxycarbonyloxy moieties described herein comprise lower hydrocarbon or substituted hydrocarbon or substituted hydrocarbon moieties.

Unless otherwise indicated, the carbamoyloxy moieties described herein are derivatives of carbamic acid in which one or both of the amine hydrogens is optionally replaced by a hydrocarbyl, substituted hydrocarbyl or heterocyclo moiety.

The terms "hydroxyl protecting group" and "hydroxy protecting group" as used herein denote a group capable of protecting a free hydroxyl group ("protected hydroxyl") which, subsequent to the reaction for which protection is employed, may be removed without disturbing the remainder of the molecule. A variety of protecting groups for the hydroxyl group and the synthesis thereof may be found in "Protective Groups in Organic Synthesis" by T. W. Greene, John Wiley and Sons, 1981, or Fieser & Fieser. Exemplary hydroxyl protecting groups include methoxymethyl, 1-ethoxyethyl, benzyloxymethyl, (.beta.-trimethylsilylethoxy)methyl, tetrahydropyranyl, 2,2,2-trichloroethoxycarbonyl, t-butyl(diphenyl)silyl, trialkylsilyl, trichloromethoxycarbonyl and 2,2,2-trichloroethoxymethyl.

As used herein, "Ac" means acetyl; "Bz" means benzoyl; "Et" means ethyl; "Me" means methyl; "Ph" means phenyl; "iPr" means isopropyl; "tBu" and "t-Bu" means tert-butyl; "R" means lower alkyl unless otherwise defined; "py" means pyridine or pyridyl; "TES" means triethylsilyl; "TMS" means trimethylsilyl; "LAH" means lithium aluminum hydride; "10-daub" means 10-desacetylbaccatin III"; "amine protecting group" includes, but is not limited to, carbamates, for example, 2,2,2-trichloroethylcarbamate or tertbutylcarbamate; "protected hydroxy" means - OP wherein P is a hydroxy protecting group; "tBuOCO" and "BOC" mean tert-butoxycarbonyl; "tAmOCO" means tert-amyloxycarbonyl; "PhCO means phenylcarbonyl"; "2-FuCO" means 2-furylcarbonyl; "2-ThCO" means 2-thienylcarbonyl; "2-PyCO" means 2-pyridylcarbonyl; "3-PyCO" means 3-pyridylcarbonyl; "4-PyCO" means 4-pyridylcarbonyl; "C₄H₇CO" means butenylcarbonyl; "EtOCO" means ethoxycarbonyl; "ibueCO" means isobutenylcarbonyl; "iBuCO" means isobutylcarbonyl; "iBuOCO" means isobutoxycarbonyl; "iPrOCO" means isopropyloxycarbonyl; "nPrOCO" means n-propyloxycarbonyl; "nPrCO" means n-propylcarbonyl; "tG₃H₅CO" means trans-propenyl carbonyl"; "ibue" means isobutenyl; "THF" means tetrahydrofuran; "DMAP" means 4-dimethylamino pyridine; "LHMDS" means Lithium HexamethylDiSilazanide.

The following examples illustrate the invention.

### Example 1

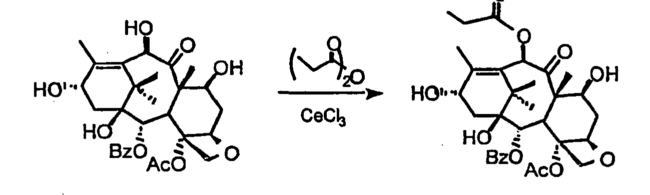

**10-Propionyl-10-deacetyl baccatin III.** To a mixture of 0.2 g (0.367 mmol) of 10-deacetyl baccatin III and 0.272 g (1.10 mmol) of CeCl₃ in 10 mL of THF at 25 °C was added 2.35 mL (18.36 mmol) of propionic anhydride. After 30 min the reaction mixture was diluted with 200 mL of EtOAc, then washed three times with 50 mL of saturated aqueous NaHCO₃ solution and brine. The organic extract was dried over Na₂SO₄ and concentrated *in vacuo.* The crude solid was purified by flash column chromatography on silica gel using 70% EtOAc/hexane as eluent to give 0.199 g (90%) of 10-propionyl-10-deacetyl baccatin III as a solid.

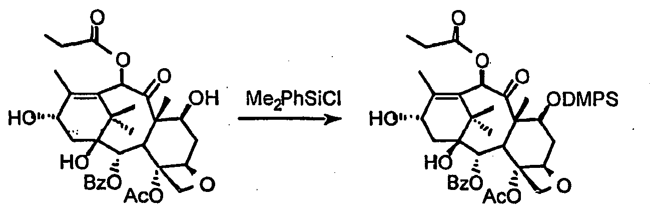

**7-Dimethylphenylsilyl-10-propionyl-10-deacetyl baccatin III.** To a solution of 0.200 g (0.333 mmol) of 10-propionyl-10-deacetyl baccatin III in 12 mL of THF at - 10°C under a nitrogen atmosphere was added dropwise 0.668 mL (4.00 mmol) of chlorodimethyl-phenylsilane and 2.48 mL (30.64 mmol) of pyridine. After 90 min the mixture was diluted with 100 mL of a 1:1 mixture of ethyl acetate and hexane. The mixture was washed with 20 mL of saturated aqueous sodium bicarbonate solution and the organic layer separated. The aqueous layer was extracted with 30 mL of a 1:1 mixture of ethyl acetate and hexane, and the combined organic extracts were washed with brine, dried over Na₂SO₄, and concentrated *in vacuo*. The crude solid was purified by flash column chromatography on silica gel using 50% EtOAc/hexane as eluent to give 0.242 g (99%) of 7-dimethylphenylsilyl-10-propionyl-10-deacetyl baccatin III as a solid.

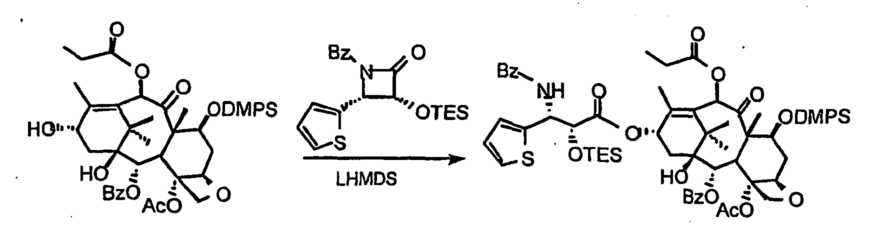

**7-Dimethylphenylsilyl-2'-O-triethylsilyl-3'-desphenyl-3'-(2-thienyl)-10-propionyl-10-deacetyl taxol**. To a solution of 0.400 g (0.544 mmol) of 7-dimethylphenylsilyl-10-propionyl-10-deacetyl baccatin III in 5.5 mL of THF at -45 °C under a nitrogen atmosphere was added 0.681 mL (0.681 mmol) of a 1M solution of LHMDS in THF. After 1 h, a solution of 0.317 g (0.818 mmol) of cis-N-benzoyl-3-triethylsilyloxy-4-(2-thienyl) azetidin-2-one in 3 mL of THF was added slowly. The mixture was warmed to 0 °C and after 3 h 10 mL of saturated aqueous sodium bicarbonate solution was added and the mixture was extracted three times with 50 mL of ethyl acetate. The combined organic extracts were washed with brine, dried over Na₂SO₄, and concentrated *in vacuo.* The crude product was purified by flash column chromatography on silica gel using 40% EtOAc/hexane as eluent to give 0.531 g (87%) of 7-dimethylphenylsilyl-2'-O-triethylsilyl-3'-desphenyl-3'-(2-thienyl)-10-propionyl-10-deacetyl taxol as a solid.

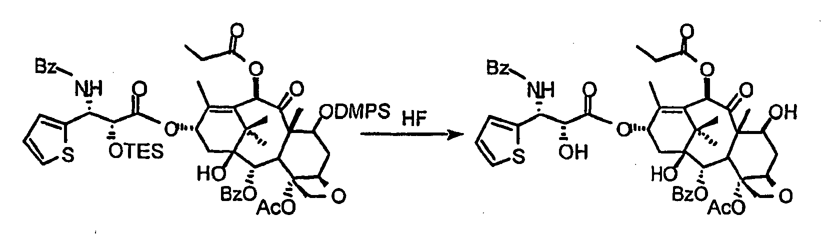

**3'-Desphenyl-3'-(2-thienyl)-10-propionyl10-deacetyl taxol**. To a solution of 0.521 g (0.464 mmol) of 7-dimethylphenylsilyl-2'-O-triethylsilyl-3'-desphenyl-3'-(2-thienyl)-10-propionyl-10-deacetyl taxol in 2 mL of CH3CN and 2 mL of pyridine at 0 °C was added 0.5 mL of a solution of 30% HF in H₂O. After 3 h 20 mL of a saturated aqueous sodium bicarbonate solution was added and the mixture was extracted three times with 50 mL of ethyl acetate. The combined organic extracts were washed with brine, dried over Na₂SO₄, and concentrated *in vacuo.* The crude product was purified by flash column chromatography on silica gel using 70% EtOAclhexane as eluent to give 0.405 g (100%) of 3'-desphenyl-3'-(2-thienyl)-10-propionyl-10-deacetyl taxol as a solid, m.p. 154-155 °C; [a]_{D}²⁵ = -45.0 (c 0.1 in CHCl3); Anal. Calcd. for C₄₆H₅₁NO₁₄S: C, 63.22; H, 5.88; Found: C, 62.94; H, 5.97.

**3'-Desphenyl-3'-(2-thienyl)-10-propionyl-10-deacetyl taxol¹H NMR data (CDCl₃)**

| **Proton** | **ppm** | **pattern** | **J (Hz)** |
|---|---|---|---|
| 2' | 4.78 | dd | H3'(2.1), 2'OH(4.1) |
| 2'OH | 3.51 | d | H2'(4.1) |
| 3' | 6.07 | dd | NH(8.6), H2'(2.1) |
| 5' | 7.04 | dd | (3.5), (5.0) |
| 1OH | 1.68 | s | |
| 2 | 5.69 | d | H3(7.0) |
| 3 | 3.85 | d | H2(7.0) |
| 4Ac | 2.42 | s | |
| 5 | 4.96 | app d | |
| 6a | 2.45-2.60 | app m | |
| 6b | 1.89 | ddd | H7(10.9), H5(2.5), H6a(14.5) |
| 7 | 4.42 | ddd | 70H(4.2), H6a(6.8), H6b(10.8) |
| 7OH | 2.45-2.60 | app m | |
| 10 | 6.32 | s | |
| 13 | 6.27 | app t | H14a,b(9.0) |
| 14a | 2.40-2.43 | app m | |
| 14b | 2.34 | dd | H14a(15.5), H13(9.0) |
| Me 16 | 1.16 | s | |
| Me 17 | 1.25 | app m | |
| Me18 | 1.84 | s | |
| Me19 | 1.70 | s | |
| 20a | 4.31 | d | H20b(8.5) |
| 20b | 4.22 | d | H20a(8.5) |
| o-benzoate | 8.14-8.16 | m | |
| o-benzamide | 7.72-7.73 | m | |
| NH | 6.88 | d | H3'(8.6) |
| CH3CH2 | 1.24 | t | CH3CH2(7.0) |
| CH3CH2 | 2.45-2.60 | app m | |

### Example 2

The procedures described in Example 1 were repeated, but other suitably protected β-lactams were substituted for the β-lactam of Example 1 to prepare the series of compounds having structural formula (13) and the combinations of substituents identified in the following table

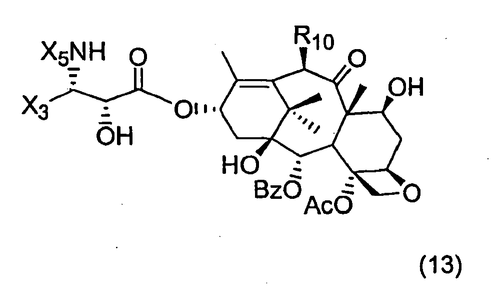

| **Compound** | **X₅** | **X₃** | **R₁₀** |
|---|---|---|---|
| 0503 | tBuOCO- | 2-pyridyl | EtCOO- |
| 0517 | tBuOCO- | 3-pyridyl | EtCOO- |
| 0521 | tBuOCO- | 4-pyridyl | EtCOO- |
| 0536 | tBuOCO- | 2-furyl | EtCOO- |
| 0549 | tBuOCO- | 3-furyl | EtCOO- |
| 0550 | tBuOCO- | 2-thienyl | EtCOO- |
| 0562 | tBuOCO- | 3-thienyl | EtCOO- |
| 0634 | PhCO- | 2-pyridyl | EtCOO- |
| 0647 | PhCO- | 3-pyridyl | EtCOO- |
| 0659 | PhCO- | 4-pyridyl | EtCOO- |
| 0663 | PhCO- | 2-furyl | EtCOO- |
| 0670 | PhCO- | 3-furyl | EtCOO- |
| 0687 | PhCO- | 2-thienyl | EtCOO- |
| 0691 | PhCO- | 3-thienyl | EtCOO- |
| 0843 | tBuOCO- | 2-furyl | cproCOO- |
| 0854 | tBuOCO- | 2-thienyl | cproCOO- |
| 0908 | PhCO- | 2-furyl | cproCOO- |
| 0919 | PhCO- | 2-thienyl | cproCOO- |
| 0951 | tBuOCO- | 2-pyridyl | PrCOO- |
| 0966 | tBuOCO- | 3-pyridyl | PrCOO- |
| 0978 | tBuOCO- | 4-pyridyl | PrCOO- |
| 0983 | tBuOCO- | 2-furyl | PrCOO- |
| 0999 | tBuOCO- | 3-furyl | PrCOO- |
| 1003 | tBuOCO- | 2-thienyl | PrCOO- |
| 1011 | tBuOCO- | 3-thienyl | PrCOO- |
| 2238 | tBuOCO- | 2-pyridyl | iPrCOO- |
| 2242 | tBuOCO- | 3-pyridyl | iPrCOO- |
| 2255 | tBuOCO- | 4-pyridyl | iPrCOO- |
| 2269 | tBuOCO- | 2-furyl | iPrCOO- |
| 2273 | tBuOCO- | 3-furyl | iPrCOO- |
| 2287 | tBuOCO- | 2-thienyl | iPrCOO- |
| 2291 | tBuOCO- | 3-thienyl | iPrCOO- |
| 2348 | tBuOCO- | 2-pyridyl | tC₃H₅COO- |
| 2353 | tBuOCO- | 3-pyridyl | tC₃H₅COO- |
| 2366 | tBuOCO- | 4-pyridyl | tC₃H₅COO- |
| 2379 | tBuOCO- | 2-furyl | tC₃H₅COO- |
| 2380 | tBuOCO- | 3-furyl | tC₃H₅COO- |
| 2392 | tBuOCO- | 2-thienyl | tC₃H₅COO- |
| 2408 | tBuOCO- | 3-thienyl | tC₃H₅COO- |
| 2464 | tBuOCO- | 2-pyridyl | ibueCOO- |
| 2472 | tBuOCO- | 4-pyridyl | ibueCOO- |
| 2488 | tBuOCO- | 2-furyl | ibueCOO- |
| 2499 | tBuOCO- | 3-furyl | ibueCOO- |
| 2503 | tBuOCO- | 2-thienyl | ibueCOO- |
| 2511 | tBuOCO- | 3-thienyl | ibueCOO- |
| 2781 | tBuOCO- | 3-furyl | cproCOO- |
| 2794 | tBuOCO- | 3-thienyl | cproCOO- |
| 2802 | tBuOCO- | 2-pyridyl | cproCOO- |
| 2813 | tBuOCO- | 4-pyridyl | cproCOO- |
| 2826 | PhCO- | 3-furyl | cproCOO- |
| 2838 | PhCO- | 3-thienyl | cproCOO- |
| 2844 | PhCO- | 2-pyridyl | cproCOO- |
| 2855 | PhCO- | 4-pyridyl | cproCOO- |
| 3053 | 2-FuCO- | 2-thienyl | EtCOO- |
| 3071 | iPrOCO- | 2-thienyl | cproCOO- |
| 3096 | EtOCO- | 2-thienyl | PrCOO- |
| 3102 | iBuOCO- | 2-furyl | cproCOO- |
| 3110 | iBuOCO- | 2-furyl | PrCOO- |
| 3129 | iBuOCO- | 2-thienyl | cproCOO- |
| 3132 | nPrCO- | 2-thienyl | cproCOO- |
| 3148 | nPrCO- | 2-thienyl | PrCOO- |
| 3163 | iBuOCO- | 2-thienyl | EtCOO- |
| 3204 | PhCO- | 2-furyl | PrCOO- |
| 3219 | nPrCO- | 2-furyl | EtCOO- |
| 3222 | nPrCO- | 2-furyl | PrCOO- |
| 3258 | PhCO- | 2-thienyl | PrCOO- |
| 3265 | iBuOCO- | 2-thienyl | PrCOO- |
| 3297 | 2-FuCO- | 2-thienyl | cproCOO- |
| 3314 | nPrCO- | 2-thienyl | PrCOO- |
| 3352 | 2-FuCO- | 2-thienyl | PrCOO- |
| 3361 | iPrOCO- | 2-thienyl | EtCOO- |
| 3370 | EtOCO- | 2-thienyl | EtCOO- |
| 3408 | 2-ThCO- | 2-thienyl | PrCOO- |
| 3417 | iPrOCO- | 2-furyl | PRCOO- |
| 3425 | 2-ThCO- | 2-thienyl | EtCOO- |
| 3453 | 2-ThCO- | 2-thienyl | cproCOO- |
| 3494 | tC₃H₅CO- | 2-thienyl | EtCOO- |
| 3513 | tC₃H₅CO- | 2-thienyl | cproCOO- |
| 3522 | iPrOCO- | 2-furyl | EtCOO- |
| 3535 | EtOCO- | 2-furyl | EtCOO- |
| 3543 | C₄H₇CO- | 2-thienyl | cproCOO- |
| 3588 | C₄H₇CO- | 2-thienyl | EtCOO- |
| 3595 | tC₃H₅CO- | 2-thienyl | PrCOO- |
| 3603 | C₄H₇CO- | 2-thienyl | PrCOO- |
| 3644 | 2-ThCO- | 2-furyl | EtCOO- |
| 3656 | 2-ThCO- | 2-furyl | PrCOO- |
| 3663 | 2-ThCO- | 2-furyl | cproCOO- |
| 3677 | EtOCO- | 2-furyl | cproCOO- |
| 3686 | 2-FuCO- | 2-furyl | PrCOO- |
| 3693 | EtOCO- | 2-furyl | PrCOO- |
| 3800 | C₄H₇CO- | 2-furyl | PrCOO- |
| 3818 | 2-FuCO- | 2-furyl | EtCOO- |
| 3853 | iPrOCO- | 2-furyl | cproCOO- |
| 3866 | 2-FuCO- | 2-furyl | cproCOO- |
| 3909 | iPrOCO- | 2-thienyl | PrCOO- |
| 3938 | C₄H₇CO- | 2-furyl | cproCOO- |
| 3945 | C₄H₇CO- | 2-furyl | EtCOO- |
| 3957 | iBuOCO- | 2-furyl | PrCOO- |
| 3971 | tC₃H₅CO- | 2-furyl | cproCOO- |
| 3982 | tC₃H₅CO- | 2-furyl | EtCOO- |
| 3994 | tC₃H₅CO- | 2-furyl | PrCOO- |
| 4051 | EtOCO- | 2-thienyl | cproCOO- |
| 4062 | nPrCO- | 2-furyl | cproCOO- |
| 4112 | 3-PyCO- | 2-thienyl | cproCOO- |
| 4121 | 3-PyCO- | 2-thienyl | EtCOO- |
| 4190 | 3-PyCO- | 2-thienyl | PrCOO- |
| 4207 | 4-PyCO- | 2-thienyl | EtCOO- |
| 4329 | ibueCO- | 2-thienyl | cproCOO- |
| 4335 | ibueCO- | 2-thienyl | EtCOO- |
| 4344 | ibueCO- | 2-thienyl | PrCOO- |
| 4665 | BuOCO- | 3-furyl | cproCOO- |
| 4704 | iBuOCO- | 3-furyl | PrCOO- |
| 4711 | iBuOCO- | 3-thienyl | EtCOO- |
| 4834 | iBuOCO- | 3-thienyl | nPrCOO- |
| 4888 | tC₃H₅CO- | 3-furyl | EtCOO- |
| 4919 | tC₃H₅CO- | 3-furyl | nPrCOO- |
| 4944 | tC₃H₅CO- | 3-furyl | cproCOO- |
| 5011 | iBuOCO- | 3-thienyl | cproCOO- |
| 5040 | tC₃H₅CO- | 3-thienyl | cproCOO- |
| 5495 | tBuOCO- | 3-furyl | EtCOO- |
| 6522 | tAmOCO- | 2-furyl | EtCOO- |

### Example 3

Following the processes described in Example 1 and elsewhere herein, the following specific taxanes having structural formula 14 may be prepared, wherein R₁₀ is as previously defined, including wherein R₁₀ is R₁₀ₐCOO- and R₁₀ₐ is (i) substituted or unsubstituted C₂ to C₈ alkyl such as ethyl, or straight, branched or cyclic propyl, butyl, pentyl, or hexyl; (ii) substituted or unsubstituted C₂ to C₈ alkenyl such as ethenyl or straight, branched or cyclic propenyl, butenyl, pentenyl or hexenyl; (iii) substituted or unsubstituted C₂ to C₈ alkynyl such as ethynyl or straight or branched propynyl, butynyl, pentynyl, or hexynyl; (iv) substituted or unsubstituted phenyl. The substituents may be those identified elsewhere herein for substituted hydrocarbyl. In one embodiment, R₁₀ may be R₁₀ₐCOO- wherein R₁₀ₐ is ethyl, straight, branched or cyclic propyl, straight or branched propenyl, or isobutenyl.

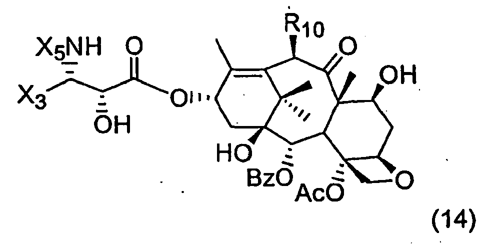

| **X₅** | **X₃** | **R₁₀** |
|---|---|---|
| tBuOCO- | 2-furyl | RₐCOO- |
| tBuOCO- | 3-furyl | RₐCOO- |
| tBuOCO- | 2-thienyl | RₐCOO- |
| tBuOCO- | 3-thienyl | RₐCOO- |
| tBuOCO- | 2-pyridyl | RₐCOO- |
| tBuOCO- | 3-pyridyl | RₐCOO- |
| tBuOCO- | 4-pyridyl | RₐCOO- |
| benzoyl | 2-furyl | RₐCOO- |
| benzoyl | 3-furyl | RₐCOO- |
| benzoyl | 2-thienyl | RₐCOO- |
| benzoyl | 3-thienyl | RₐCOO- |
| benzoyl | 2-pyridyl | RₐCOO- |
| benzoyl | 3-pyridyl | RₐCOO- |
| benzoyl | 4-pyridyl | RₐCOO- |
| 2-FuCO- | 2-furyl | RₐCOO- |
| 2-FuCO- | 3-furyl | RₐCOO- |
| 2-FuCO- | 2-thienyl | RₐCOO- |
| 2-FuCO- | 3-thienyl | RₐCOO- |
| 2-FuCO- | 2-pyridyl | RₐCOO- |
| 2-FuCO- | 3-pyridyl | RₐCOO- |
| 2-FuCO- | 4-pyridyl | RₐCOO- |
| 2-ThCO- | 2-furyl | RₐCOO- |
| 2-ThCO- | 3-furyl | RₐCOO- |
| 2-ThCO- | 2-thienyl | RₐCOO- |
| 2-ThCO- | 3-thienyl | RₐCOO- |
| 2-ThCO- | 2-pyridyl | RₐCOO- |
| 2-ThCO- | 3-pyridyl | RₐCOO- |
| 2-ThCO- | 4-pyridyl | RₐCOO- |
| 2-PyCO- | 2-furyl | RₐCOO- |
| 2-PyCO- | 3-furyl | RₐCOO- |
| 2-PyCO- | 2-thienyl | RₐCOO- |
| 2-PyCO- | 3-thienyl | RₐCOO- |
| 2-PyCO- | 2-pyridyl | RₐCOO- |
| 2-PyCO- | 3-pyridyl | RₐCOO- |
| 2-PyCO- | 4-pyridyl | RₐCOO- |
| 3-PyCO- | 2-furyl | RₐCOO- |
| 3-PyCO- | 3-furyl | RₐCOO- |
| 3-PyCO- | 2-thienyl | RₐCOO- |
| 3-PyCO- | 3-thienyl | RₐCOO- |
| 3-PyCO- | 2-pyridyl | RₐCOO- |
| 3-PyCO- | 3-pyridyl | RₐCOO- |
| 3-PyCO- | 4-pyridyl | RₐCOO- |
| 4-PyCO- | 2-furyl | RₐCOO- |
| 4-PyCO- | 3-furyl | RₐCOO- |
| 4-PyCO- | 2-thienyl | RₐCOO- |
| 4-PyCO- | 3-thienyl | RₐCOO- |
| 4-PyCO- | 2-pyridyl | RₐCOO- |
| 4-PyCO- | 3-pyridyl | RₐCOO- |
| 4-PyCO- | 4-pyridyl | RₐCOO- |
| C₄H₇CO- | 2-furyl | RₐCOO- |
| C₄H₇CO- | 3-furyl | RₐCOO- |
| C₄H₇CO- | 2-thienyl | RₐCOO- |
| C₄H₇CO- | 3-thienyl | RₐCOO- |
| C₄H₇CO- | 2-pyridyl | RₐCOO- |
| C₄H₇CO- | 3-pyridyl | RₐCOO- |
| C₄H₇CO- | 4-pyridyl | RₐCOO- |
| EtOCO- | 2-furyl | RₐCOO- |
| EtOCO- | 3-furyl | RₐCOO- |
| EtOCO- | 2-thienyl | RₐCOO- |
| EtOCO- | 3-thienyl | RₐCOO- |
| EtOCO- | 2-pyridyl | RₐCOO- |
| EtOCO- | 3-pyridyl | RₐCOO- |
| EtOCO- | 4-pyridyl | RₐCOO- |
| ibueCO- | 2-furyl | RₐCOO- |
| ibueCO- | 3-furyl | RₐCOO- |
| ibueCO- | 2-thienyl | RₐCOO- |
| ibueCO- | 3-thienyl | RₐCOO- |
| ibueCO- | 2-pyridyl | RₐCOO- |
| ibueCO- | 3-pyridyl | RₐCOO- |
| ibueCO- | 4-pyridyl | RₐCOO- |
| iBuCO- | 2-furyl | RₐCOO- |
| iBuCO- | 3-furyl | RₐCOO- |
| iBuCO- | 2-thienyl | RₐCOO- |
| iBuCO- | 3-thienyl | RₐCOO- |
| iBuCO- | 2-pyridyl | RₐCOO- |
| iBuCO- | 3-pyridyl | RₐCOO- |
| iBuCO- | 4-pyridyl | RₐCOO- |
| iBuOCO- | 2-furyl | COO- |
| iBuOCO- | 3-furyl | RₐCOO- |
| iBuOCO- | 2-thienyl | RₐCOO- |
| iBuOCO- | 3-thienyl | RₐCOO- |
| iBuOCO- | 2-pyridyl | RₐCOO- |
| iBuOCO- | 3-pyridyl | RₐCOO- |
| iBuOCO- | 4-pyridyl | RₐCOO- |
| iPrOCO- | 2-furyl | RₐCOO- |
| iPrOCO- | 3-furyl | RₐCOO- |
| iPrOCO- | 2-thienyl | RₐCOO- |
| iPrOCO- | 3-thienyl | RₐCOO- |
| iPrOCO- | 2-pyridyl | RₐCOO- |
| iPrOCO- | 3-pyridyl | RₐCOO- |
| iPrOCO- | 4-pyridyl | RₐCOO- |
| nPrOCO- | 2-furyl | RₐCOO- |
| nPrOCO- | 3-furyl | RₐCOO- |
| nPrOCO- | 2-thienyl | RₐCOO- |
| nPrOCO- | 3-thienyl | RₐCOO- |
| nPrOCO- | 2-pyridyl | RₐCOO- |
| nPrOCO- | 3-pyridyl | RₐCOO- |
| nPrOCO- | 4-pyridyl | RₐCOO- |
| nPrCO- | 2-furyl | RₐCOO- |
| nPrCO- | 3-furyl | RₐCOO- |
| nPrCO- | 2-thienyl | RₐCOO- |
| nPrCO- | 3-thienyl | RₐCOO- |
| nPrCO- | 2-pyridyl | RₐCOO- |
| nPrCO- | 3-pyridyl | RₐCOO- |
| nPrCO- | 4-pyridyl | RₐCOO- |
| 2-FuCO- | 3-furyl | EtCOO- |
| 2-FuCO- | 3-thienyl | EtCOO- |
| 2-FuCO- | 2-pyridyl | EtCOO- |
| 2-FuCO- | 3-pyridyl | EtCOO- |
| 2-FuCO- | 4-pyridyl | EtCOO- |
| 2-ThCO- | 3-furyl | EtCOO- |
| 2-ThCO- | 3-thienyl | EtCOO- |
| 2-ThCO- | 2-pyridyl | EtCOO- |
| 2-ThCO- | 3-pyridyl | EtCOO- |
| 2-ThCO- | 4-pyridyl | EtCOO- |
| 2-PyCO- | 2-furyl | EtCOO- |
| 2-PyCO- | 3-furyl | EtCOO- |
| 2-PyCO- | 2-thienyl | EtCOO- |
| 2-PyCO- | 3-thienyl | EtCOO- |
| 2-PyCO- | 2-pyridyl | EtCOO- |
| 2-PyCO- | 3-pyridyl | EtCOO- |
| 2-PyCO- | 4-pyridyl | EtCOO- |
| 3-PyCO- | 2-furyl | EtCOO- |
| 3-PyCO- | 3-furyl | EtCOO- |
| 3-PyCO- | 3-thienyl | EtCOO- |
| 3-PyCO- | 2-pyridyl | EtCOO- |
| 3-PyCO- | 3-pyridyl | EtCOO- |
| 3-PyCO- | 4-pyridyl | EtCOO- |
| 4-PyCO- | 2-furyl | EtCOO- |
| 4-PyCO- | 3-furyl | EtCOO- |
| 4-PyCO- | 3-thienyl | EtCOO- |
| 4-PyCO- | 2-pyridyl | EtCOO- |
| 4-PyCO- | 3-pyridyl | EtCOO- |
| 4-PyCO- | 4-pyridyl | EtCOO- |
| C₄H₇CO- | 3-furyl | EtCOO- |
| C₄H₇CO- | 3-thienyl | EtCOO- |
| C₄H₇CO- | 2-pyridyl | EtCOO- |
| C₄H₇CO- | 3-pyridyl | EtCOO- |
| C₄H₇CO- | 4-pyridyl | EtCOO- |
| EtOCO- | 3-furyl | EtCOO- |
| EtOCO- | 3-thienyl | EtCOO- |
| EtOCO- | 2-pyridyl | EtCOO- |
| EtOCO- | 3-pyridyl | EtCOO- |
| EtOCO- | 4-pyridyl | EtCOO- |
| ibueCO- | 2-furyl | EtCOO- |
| ibueCO- | 3-furyl | EtCOO- |
| ibueCO- | 3-thienyl | EtCOO- |
| ibueCO- | 2-pyridyl | EtCOO- |
| ibueCO- | 3-pyridyl | EtCOO- |
| ibueCO- | 4-pyridyl | EtCOO- |
| iBuCO- | 2-furyl | EtCOO- |
| iBuCO- | 3-furyl | EtCOO- |
| iBuCO- | 2-thienyl | EtCOO- |
| iBuCO- | 3-thienyl | EtCOO- |
| iBuCO- | 2-pyridyl | EtCOO- |
| iBuCO- | 3-pyridyl | EtCOO- |
| iBuCO- | 4-pyridyl | EtCOO- |
| iBuOCO- | 2-furyl | EtCOO- |
| iBuOCO- | 2-pyridyl | EtCOO- |
| iBuOCO- | 3-pyridyl | EtCOO- |
| iBuOCO- | 4-pyridyl | EtCOO- |
| iPrOCO- | 3-furyl | EtCOO- |
| iPrOCO- | 3-thienyl | EtCOO- |
| iPrOCO- | 2-pyridyl | EtCOO- |
| iPrOCO- | 3-pyridyl | EtCOO- |
| iPrOCO- | 4-pyridyl | EtCOO- |
| nPrOCO- | 2-furyl | EtCOO- |
| nPrOCO- | 3-furyl | EtCOO- |
| nPrOCO- | 2-thienyl | EtCOO- |
| nPrOCO- | 3-thienyl | EtCOO- |
| nPrOCO- | 2-pyridyl | EtCOO- |
| nPrOCO- | 3-pyridyl | EtCOO- |
| nPrOCO- | 4-pyridyl | EtCOO- |
| nPrCO- | 3-furyl | EtCOO- |
| nPrCO- | 3-thienyl | EtCOO- |
| nPrCO- | 2-pyridyl | EtCOO- |
| nPrCO- | 3-pyridyl | EtCOO- |
| nPrCO- | 4-pyridyl | EtCOO- |

### Example 4

Following the processes described in Example 1 and elsewhere herein, the following specific taxanes having structural formula 15 may be prepared, wherein R₇ is hydroxy and R₁₀ in each of the series (that is, each of series "A" through "K") is as previously defined, including wherein R₁₀ is R₁₀ₐCOO- and R₁₀ₐ is (i) substituted or unsubstituted, preferably unsubstituted, C₂ to C₈ alkyl (straight, branched or cyclic), such as ethyl, propyl, butyl, pentyl, or hexyl; (ii) substituted or unsubstituted, preferably unsubstituted, C₂ to C₈ alkenyl (straight, branched or cyclic), such as ethenyl, propenyl, butenyl, pentenyl or hexenyl; (iii) substituted or unsubstituted, preferably unsubstituted, C₂ to C₈ alkynyl (straight or branched) such as ethynyl, propynyl, butynyl, pentynyl, or hexynyl; (iv) substituted or unsubstituted, preferably unsubstituted, or phenyl.

In the "A" series of compounds, X₁₀ is as otherwise as defined herein. Preferably, heterocyclo is substituted or unsubstitued furyl, thienyl, or pyridyl, X₁₀ is substituted or unsubstitued furyl, thienyl, pyridyl, phenyl, or lower alkyl (e.g., tert-butyl), and R₇ and R₁₀ each have the beta stereochemical configuration.

In the "B" series of compounds, X₁₀ and R₂ₐ are as otherwise as defined herein. Preferably, heterocyclo is preferably substituted or unsubstitued furyl, thienyl, or pyridyl, X₁₀ is preferably substituted or unsubstitued furyl, thienyl, pyridyl, phenyl, or lower alkyl (e.g., tert-butyl), R₂ₐ is preferably substituted or unsubstitued furyl, thienyl, pyridyl, phenyl, or lower alkyl, and R₇ and R₁₀ each have the beta stereochemical configuration.

In the "C" series of compounds, X₁₀ and R₉ₐ are as otherwise as defined herein. Preferably, heterocyclo is preferably substituted or unsubstitued furyl, thienyl, or pyridyl, X₁₀ is preferably substituted or unsubstitued furyl, thienyl, pyridyl, phenyl, or lower alkyl (e.g., tert-butyl), R₉ₐ is preferably substituted or unsubstitued furyl, thienyl, pyridyl, phenyl, or lower alkyl, and R₇, R₉ and R₁₀ each have the beta stereochemical configuration.

In the "D" and "E" series of compounds, X₁₀ is as otherwise as defined herein. Preferably, heterocyclo is preferably substituted or unsubstitued furyl, thienyl, or pyridyl, X₁₀ is preferably substituted or unsubstitued furyl, thienyl, pyridyl, phenyl, or lower alkyl (e.g., tert-butyl), and R₇, R₉ (series D only) and R₁₀ each have the beta stereochemical configuration.

In the "F" series of compounds, X₁₀, R₂ₐ and R₉ₐ are as otherwise as defined herein. Preferably, heterocyclo is preferably substituted or unsubstitued furyl, thienyl, or pyridyl, X₁₀ is preferably substituted or unsubstitued furyl, thienyl, pyridyl, phenyl, or lower alkyl (e.g., tert-butyl), R₂ₐ is preferably substituted or unsubstitued furyl, thienyl, pyridyl, phenyl, or lower alkyl, and R₇, R₉ and R₁₀ each have the beta stereochemical configuration.

In the "G" series of compounds, X₁₀ and R₂ₐ are as otherwise as defined herein. Preferably, heterocyclo is preferably substituted or unsubstitued furyl, thienyl, or pyridyl, X₁₀ is preferably substituted or unsubstitued furyl, thienyl, pyridyl, phenyl, or lower alkyl (e.g., tert-butyl), R₂ₐ is preferably substituted or unsubstitued furyl, thienyl, pyridyl, phenyl, or lower alkyl, and R₇, R₉ and R₁₀ each have the beta stereochemical configuration.

In the "H" series of compounds, X₁₀ is as otherwise as defined herein. Preferably, heterocyclo is preferably substituted or unsubstitued furyl, thienyl, or pyridyl, X₁₀ is preferably substituted or unsubstitued furyl, thienyl, pyridyl, phenyl, or lower alkyl (e.g., tert-butyl), R₂ₐ is preferably substituted or unsubstitued furyl, thienyl, pyridyl, phenyl, or lower alkyl, and R₇ and R₁₀ each have the beta stereochemical configuration.

In the "I" series of compounds, X₁₀ and R₂ₐ are as otherwise as defined herein. Preferably, heterocyclo is preferably substituted or unsubstitued furyl, thienyl, or pyridyl, X₁₀ is preferably substituted or unsubstitued furyl, thienyl, pyridyl, phenyl, or lower alkyl (e.g., tert-butyl), R₂ₐ is preferably substituted or unsubstitued furyl, thienyl, pyridyl, phenyl, or lower alkyl, and R₇ and R₁₀ each have the beta stereochemical configuration.

In the "J" series of compounds, X₁₀ and R₂ₐ are as otherwise as defined herein. Preferably, heterocyclo is preferably substituted or unsubstitued furyl, thienyl, or pyridyl, X₁₀ is preferably substituted or unsubstitued furyl, thienyl, pyridyl, phenyl, or lower alkyl (e.g., tert-butyl), R₂ₐ is preferably substituted or unsubstitued furyl, thienyl, pyridyl, phenyl, or lower alkyl, and R₇, R₉ and R₁₀ each have the beta stereochemical configuration.

In the "K" series of compounds, X₁₀, R₂ₐ and R₉ₐ are as otherwise as defined herein. Preferably, heterocyclo is preferably substituted or unsubstitued furyl, thienyl, or pyridyl, X₁₀ is preferably substituted or unsubstitued furyl, thienyl, pyridyl, phenyl, or lower alkyl (e.g., tert-butyl), R₂ₐ is preferably substituted or unsubstitued furyl, thienyl, pyridyl, phenyl, or lower alkyl, and R₇, R₉ and R₁₀ each have the beta stereochemical configuration.

Any substituents of each X₃, X₅, R₂, R₉, R₁₀ may be hydrocarbyl or any of the heteroatom containing substituents selected from the group consisting of heterocyclo, alkoxy, alkenoxy, alkynoxy, aryloxy, hydroxy, protected hydroxy, keto, acyloxy, nitro, amino, amido, thiol, ketal, acetal, ester and ether moieties, but not phosphorous containing moieties.

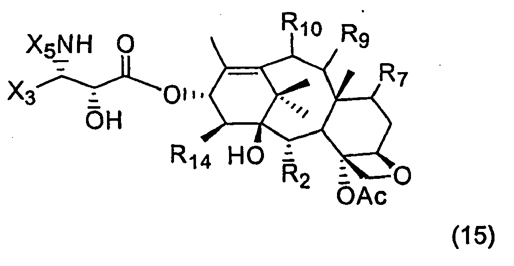

| **Series** | **X₅** | **X₃** | **R₁₀** | **R₂** | **R₉** | **R₁₄** |
|---|---|---|---|---|---|---|
| A1 | -COOX₁₀ | heterocyclo | R₁₀ₐCOO- | C₆H₅COO- | O | H |
| A2 | -COX₁₀ | heterocyclo | R₁₀ₐCOO- | C₆H₅COO- | O | H |
| A3 | -CONHX₁₀ | heterocyclo | R₁₀ₐCOO- | C₆H₅COO- | O | H |
| B1 | -COOX₁₀ | heterocyclo | R₁₀ₐCOO- | R₂ₐCOO- | O | H |
| B2 | -COX₁₀ | heterocyclo | R₁₀aCOO- | R₂ₐCOO- | O | H |
| B3 | -CONHX₁₀ | heterocyclo | R₁₀ₐCOO- | R₂ₐCOO- | O | H |
| C1 | -COOX₁₀ | heterocyclo | R₁₀ₐCOO- | C₆H₅COO- | R₉ₐCOO- | H |
| C2 | -COX₁₀ | heterocyclo | R₁₀ₐCOO- | C₆H₅COO- | R₉ₐCOO- | H |
| C3 | -CONHX₁₀ | heterocyclo | R₁₀ₐCOO- | C₆H₅COO- | R₉ₐCOO- | H |
| D1 | -CCOOX₁₀ | heterocyclo | R₁₀ₐCOO- | C₆H₅COO- | OH | H |
| D2 | -COX₁₀ | heterocyclo | R₁₀ₐCOO- | C₆H₅COO- | OH | H |
| D3 | -CONHX₁₀ | heterocyclo | R₁₀.COO- | C₆H₅COO- | OH | H |
| E1 | -COOX₁₀ | heterocyclo | R₁₀ₐCOO- | C₆H₅COO- | O | OH |
| E2 | -COX₁₀ | heterocyclo | R₁₀ₐCOO | C₆H₅COO- | O | OH |
| E3 | -CONHX₁₀ | heterocyclo | R₁₀ₐCOO- | C₆H5COO- | O | OH |
| F1 | -COOX₁₀ | heterocyclo | R₁₀ₐCOO- | R₂ₐCOO- | R₉ₐCOO- | H |
| F2 | -COX₁₀ | heterocyclo | R₁₀ₐCOO- | R₂ₐCOO- | R₉ₐCOO- | H |
| F3 | -CONHX₁₀ | heterocyclo | R₁₀ₐCOO- | R₂ₐCOO- | R₉ₐCOO- | H |
| G1 | -COOX₁₀ | heterocyclo | R₁₀ₐCOO- | R₂ₐCOO- | OH | H |
| G2 | -COX₁₀ | heterocyclo | R₁₀ₐCOO- | R₂ₐCOO- | OH | H |
| G3 | -CONHX₁₀ | heterocyclo | R₁₀ₐOO- | R₂ₐCOO- | OH | H |
| H1 | -COOX₁₀ | heterocyclo | R₁₀ₐCOO- | C₆H₅COO- | OH | OH |
| H2 | -COX₁₀ | heterocyclo | R₁₀ₐCOO- | C₆H₅COO- | OH | OH |
| H3 | -CONHX₁₀ | heterocyclo | R₁₀ₐCOO- | C₆H₅COO- | OH | OH |
| I1 | -COOX₁₀ | heterocyclo | R₁₀ₐCOO- | R₂ₐCOO- | O | OH |
| I2 | -COX₁₀ | heterocyclo | R₁₀ₐCOO- | R₂ₐCOO- | O | OH |
| I3 | -CONHX₁₀ | heterocyclo | R₁₀ₐCOO- | R₂ₐCOO- | O | OH |
| J1 | -COOX₁₀ | heterocyclo | R₁₀ₐOO- | R₂ₐCOO- | OH | OH |
| J2 | -COX₁₀ | heterocyclo | R₁₀ₐCOO- | R₂ₐCOO- | OH | OH |
| J3 | -CONHX₁₀ | heterocyclo | R₁₀ₐCOO- | R₂ₐCOO- | OH | OH |
| K1 | -COOX₁₀ | heterocyclo | R₁₀ₐCOO- | R₂ₐCOO- | F₉ₐCOO- | OH |
| K2 | -COX₁₀ | heterocyclo | R₁₀aCOO- | R₂ₐCOO- | R₉ₐCOO- | OH |
| K3 | -CONHX₁₀ | heterocyclo | R₁₀ₐCOO- | R₂ₐCOO- | R₉ₐCOO- | OH |

### Example 5

### In Vitro cytotoxicity measured by the cell colony formation assay

Four hundred cells (HCT116) were plated in 60 mm Petri dishes containing 2.7 mL of medium (modified McCoy's 5a medium containing 10% fetal bovine serum and 100 units/mL penicillin and 100 g/mL streptomycin). The cells were incubated in a CO₂ incubator at 37 °C for 5 h for attachment to the bottom of Petri dishes. The compounds identified in Example 2 were made up fresh in medium at ten times the final concentration, and then 0.3 mL of this stock solution was added to the 2.7 mL of medium in the dish. The cells were then incubated with drugs for 72 h at 37 ° C. At the end of incubation the drug-containing media were decanted, the dishes were rinsed with 4 mL of Hank's Balance Salt Solution (HBSS), 5 mL of fresh medium was added, and the dishes were returned to the incubator for colony formation. The cell colonies were counted using a colony counter after incubation for 7 days. Cell survival was calculated and the values of ID50 (the drug concentration producing 50% inhibition of colony formation) were determined for each tested compound.

| **Compound** | **IN VITRO** **ID 50 (nm) HCT116** |
|---|---|
| taxol | 2.1 |
| docetaxel | 0.6 |
| 0503 | <1 |
| 0517 | <10 |
| 0521 | <1 |
| 0536 | <1 |
| 0549 | <10 |
| 0550 | <10 |
| 0562 | <1 |
| 0634 | <10 |
| 0647 | 12.0 |
| 0659 | <1 |
| 0663 | <1 |
| 0670 | <1 |
| 0687 | <1 |
| 0691 | <1 |
| 0843 | <1 |
| 0854 | <1 |
| 0908 | <1 |
| 0919 | <1 |
| 0951 | <1 |
| 0966 | <10 |
| 0978 | <1 |
| 0983 | <1 |
| 0999 | <1 |
| 1003 | <1 |
| 1011 | <1 |
| 2238 | <1 |
| 2242 | <10 |
| 2255 | <1 |
| 2269 | <1 |
| 2273 | <1 |
| 2287 | <1 |
| 2291 | <1 |
| 2348 | <1 |
| 2353 | <10 |
| 2366 | <1 |
| 2379 | <1 |
| 2380 | <1 |
| 2392 | <1 |
| 2408 | <1 |
| 2464 | <1 |
| 2472 | <1 |
| 2488 | <1 |
| 2499 | <1 |
| 2503 | <1 |
| 2511 | <1 |
| 2781 | <1 |
| 2794 | <1 |
| 2802 | <1 |
| 2813 | <1 |
| 2826 | <1 |
| 2838 | <1 |
| 2844 | <10 |
| 2855 | <1 |
| 3053 | <1 |
| 3071 | <1 |
| 3096 | <1 |
| 3102 | <1 |
| 3110 | <1 |
| 3129 | <10 |
| 3132 | <1 |
| 3148 | <1 |
| 3163 | <1 |
| 3204 | <1 |
| 3219 | <1 |
| 3222 | <1 |
| 3258 | <1 |
| 3265 | <10 |
| 3297 | <1 |
| 3314 | <1 |
| 3352 | <1 |
| 3361 | <1 |
| 3370 | <1 |
| 3408 | <1 |
| 3417 | <1 |
| 3425 | <1 |
| 3453 | <1 |
| 3494 | <1 |
| 3513 | <1 |
| 3522 | <1 |
| 3535 | <1 |
| 3543 | <10 |
| 3588 | <10 |
| 3595 | <1 |
| 3603 | <10 |
| 3644 | <1 |
| 3656 | <1 |
| 3663 | <1 |
| 3677 | <1 |
| 3686. | <1 |
| 3693 | <1 |
| 3800 | <1 |
| 3818 | <1 |
| 3853 | <1 |
| 3866 | <1 |
| 3909 | <1 |
| 3938 | <10 |
| 3945 | <1 |
| 3957 | <10 |
| 3971 | <1 |
| 3982 | <1 |
| 3994 | <1 |
| 4051 | <1 |
| 4062 | <1 |
| 4112 | <10 |
| 4121 | <10 |
| 4190 | <10 |
| 4207 | <10 |
| 4329 | <1 |
| 4335 | <1 |
| 4344 | <1 |
| 4665 | <10 |
| 4704 | <10 |
| 4711 | <10 |
| 4834 | <10 |
| 4888 | <1 |
| 4919 | <1 |
| 4944 | <1 |
| 5011 | <10 |
| 5040 | <1 |
| 5495 | <1 |
| 6522 | <1 |

### Example 6

### Preparation of Solutions for Oral Administration

Antitumor compound 0550 was dissolved in ethanol to form a solution containing 140 mg of the compound per of solution. An equal volume of Cremophor® EL solution was added to the solution while stirring to form a solution containing 70 mg of compound 0550 per ml of solution. This solution was diluted using 9 parts by weight of saline to form a pharmaceutically acceptable solution for administration to a patient.

## Claims

1. A taxane having the formula 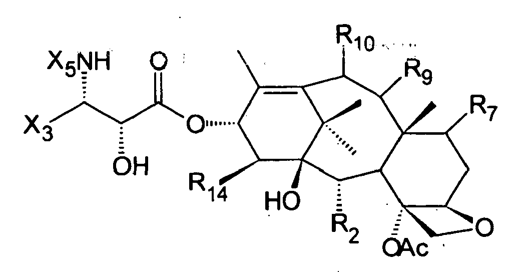
R₂ is acyloxy;
R₇ is hydroxy;
R₉ is keto, hydroxy, or acyloxy;
R₁₀ is R₁₀ₐCOO-;
R₁₀ₐ is hydrocarbyl or substituted hydrocarbyl, wherein said hydrocarbyl or substituted hydrocarbyl contains carbon atoms in the alpha and beta positions relative to the carbon atom of which R₁₀ₐ is a substituent;
R₁₄ is hydrogen or hydroxy;
X₃ is heterocyclo;
X₅ is -COX₁₀, -COOX,₁₀, or -CONHX₁₀;
X₁₀ is hydrocarbyl, substituted hydrocarbyl, or heterocyclo; and
Ac is acetyl.

2. The taxane of claim 1, wherein R₁₀ₐ is C₂ - C₈ alkyl.

3. The taxane of claim 2, wherein X₃ is 2-furyl, 3-furyl, 2-thienyl, 3-thienyl, 2-pyridyl, 3-pyridyl, or 4-pyridyl.

4. The taxane of claim 2, wherein X₅ is -COX₁₀ and X₁₀ is substituted or unsubstituted phenyl, 2-furyl, 3-furyl, 2-thienyl, 3-thienyl, 2-pyridyl, 3-pyridyl, 4-pyridyl, C₁ - C₈ alkyl, C₂ - C₈ alkenyl, or C₂ - C₈ alkynyl, or X₅ is -COOX₁₀ and X₁₀ is substituted or unsubstituted C₁ - C₈ alkyl, C₂ - C₈ alkenyl, or C₂ - C₈ alkynyl.

5. The taxane of any one of claims 2 to 4, wherein R₁₄ is hydrogen.

6. The taxane of any one of claims 2 to 4, wherein R₂ is benzoyloxy.

7. The taxane of any one of claims 2 to 4, wherein R₁₄ is hydogen and R₉ is keto.

8. The taxane of any one of claims 2 to 4, wherein R₂ is benzoyloxy and R₉ is keto.

9. The taxane of any one of claims 2 to 4, wherein R₁₄ is hydrogen and R₂ is benzoyloxy.

10. The taxane of any one of claims 2 to 4, wherein R₁₄ is hydrogen, R₉ is keto, and R₂ is benzoyloxy.

11. The taxane of claim 1, wherein R₁₀ₐ is ethyl.

12. The taxane of claim 11, wherein X₃ is 2-furyl, 3-furyl, 2-thienyl, 3-thienyl, 2-pyridyl, 3-pyridyl, or 4-pyridyl.

13. The taxane of claim 11, wherein X₅ is -COX₁₀ and X₁₀ is substituted or unsubstituted phenyl, 2-furyl, 3-furyl, 2-thienyl, 3-thienyl, 2-pyridyl, 3-pyridyl, 4-pyridyl, C₁ - C₈ alkyl, C₂ - C₈ alkenyl, or C₂ - C₈ alkynyl, or X₅ is -COOX₁₀ and X₁₀ is substituted or unsubstituted C₁ - C₈ alkyl, C₂ - C₈ alkenyl, or C₂ - C₈ alkynyl.

14. The taxane of any one of claims 11 to 13, wherein R₁₄ is hydrogen.

15. The taxane of any one of claims 11 to 13, wherein R₂ is benzoyloxy.

16. The taxane of any one of claims 11 to 13, wherein R₁₄ is hydrogen and R₉ is keto.

17. The taxane of any one of claims 11 to 13, wherein R₂ is benzoyloxy and R₉ is keto.

18. The taxane of any one of claims 11 to 13, wherein R₁₄ is hydrogen and R₂ is benzoyloxy.

19. The taxane of any one of claims 11 to 13, wherein R₁₄ is hydrogen, R₉ is keto, and R₂ is benzoyloxy.

20. A taxane of claim 1 having the formula 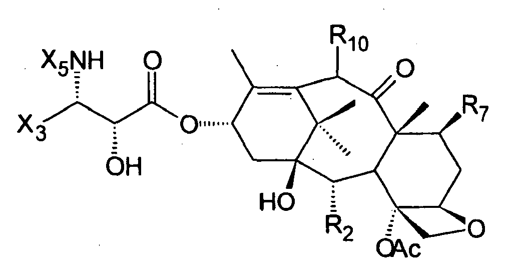
R₂ is benzoyloxy;
R₇ is hydroxy;
R₁₀ is R₁₀ₐCOO-;
X₃ is heterocyclo;
X₅ is -COX₁₀, -COOX₁₀, or -CONHX₁₀;
X₁₀ is hydrocarbyl, substituted hydrocarbyl, or heterocyclo; and
R₁₀ₐ is hydrocarbyl or substituted hydrocarbyl, wherein said hydrocarbyl or substituted hydrocarbyl contains carbon atoms in the alpha and beta positions relative to the carbon atom of which R₁₀, is a substituent; and
Ac is acetyl.

21. The taxane of claim 20, wherein X₃ is 2-furyl, 3-furyl, 2-thienyl, 3-thienyl, 2-pyridyl, 3-pyridyl, or 4-pyridyl.

22. The taxane of claim 20, wherein X₅ is -COX₁₀ and X₁₀ is substituted or unsubstituted phenyl, 2-furyl, 3-furyl, 2-thienyl, 3-thienyl, 2-pyridyl, 3-pyridyl, 4-pyridyl, C₁ - C₈ alkyl, C₂ - C₈ alkenyl, or C₂ - C₈ alkynyl, or X₅ is -COOX₁₀ and X₁₀ is substituted or unsubstituted C₁ - C₈ alkyl, C₂ - C₈ alkenyl, or C₂ - C₈ alkynyl.

23. The taxane of any one of claims 20 to 22, wherein X₃ is furyl or thienyl.

24. The taxane of any one of claims 20 to 22, wherein R₁₀ₐ is ethyl or propyl.

25. The taxane of claim 20, wherein X₃ is furyl or thienyl, R₁₀ₐ is ethyl, and X₅ is -COX₁₀ and X₁₀ is phenyl, or X₅ is -COOX₁₀ and X₁₀ is t-butyl.

26. The taxane of claim 20, wherein X₃ is 2-furyl or 2-thienyl, R₁₀ₐ is ethyl, X₅ is -COOX₁₀ and X₁₀ is t-butyl.

27. The taxane of claim 20, wherein X₅ is -COOX₁₀ and X₁₀ is t-butyl, R₁₀ₐ is ethyl, propyl, isopropyl, or trans-propenyl, and X₃ is 2-furyl or 3-furyl.

28. The taxane of claim 20, wherein X₅ is -COOX₁₀ and X₁₀ is t-butyl, R₁₀ₐ is ethyl, propyl, isopropyl, or trans-propenyl, and X₃ is 2-thienyl or 3-thienyl.

29. The taxane of claim 20, wherein X₅ is -COX₁₀ and X₁₀ is phenyl, R₁₀ₐ is ethyl, and X₃ is 2-pyridyl, 3-pyridyl, 4-pyridyl, 2-furyl, 3-furyl, 2-thienyl, or 3-thienyl.

30. The taxane of claim 20, wherein X₅ is -COOX₁₀ and X₁₀ is ethyl, R₁₀ₐ is ethyl, cyclopropyl, or propyl, and X₃ is 2-thienyl or 2-furyl.

31. The taxane of claim 20, wherein X₅ is -COX₁₀ and X₁₀ is 2-furyl or 2-thienyl, R₁₀ₐ is ethyl, cyclopropyl, or propyl, and X₃ is 2-thienyl.

32. The taxane of claim 20, wherein X₅ is -COOX₁₀ and X₁₀ is t-butyl, R₁₀ₐ is cyclopropyl, and X₃ is 2-furyl, 3-furyl, 2-thienyl, or 3-thienyl.

33. The taxane of claim 20, wherein X₅ is -COOX₁₀ and X₁₀ is isobutyl, R₁₀ₐ is cyclopropyl or propyl, and X₃ is 2-furyl or 3-furyl.

34. The taxane of claim 20, wherein X₅ is -COOX₁₀ and X₁₀ is isobutyl, R₁₀ₐ is cyclopropyl, propyl, or ethyl, and X₃ is 2-thienyl.

35. The taxane of claim 20, wherein X₅ is -COX₁₀ and X₁₀ is trans-propenyl, isobutenyl, or butenyl, R₁₀ₐ is propyl, cyclopropyl, or ethyl, and X₃ is 2-thienyl.

36. The taxane of claim 20, wherein X₅ is -COX₁₀ and X₁₀ is butenyl or trans-propenyl, R₁₀ₐ is propyl, cyclopropyl, or ethyl, and X₃ is 2-furyl.

37. The taxane of claim 20, wherein X₅ is -COX₁₀ and X₁₀ is trans-propenyl, R₁₀ₐ is n-propyl, cyclopropyl, or ethyl, and X₃ is 3-furyl.

38. The taxane of claim 20, wherein X₅ is -COX₁₀ and X₁₀ is phenyl, R₁₀ₐ is cyclopropyl, and X₃ is 2-pyridyl, 4-pyridyl, 2-furyl, 3-furyl, 2-thienyl, or 3-thienyl.

39. The taxane of claim 20, wherein X₅ is -COX₁₀ and X₁₀ is n-propyl, R₁₀ₐ is cyclopropyl, ethyl, or propyl, and X₃ is 2-furyl.

40. The taxane of claim 20, wherein X₅ is -COX₁₀ and X₁₀ is n-propyl, R₁₀ₐ is cyclopropyl or propyl, and X₃ is 2-thienyl.

41. The taxane of claim 20, wherein X₅ is -COOX₁₀ and X₁₀ is isobutyl, R₁₀ₐ is propyl, and X₃ is 2-furyl.

42. The taxane of claim 20, wherein X₅ is -COOX₁₀ and X₁₀ is isobutyl, R₁₀ₐ is n-propyl, cyclopropyl, or ethyl, and X₃ is 3-thienyl.

43. The taxane of claim 20, wherein X₅ is -COOX₁₀ and X₁₀ is isopropyl, R₁₀ₐ is ethyl, cyclopropyl, or propyl, and X₃ is 2-thienyl or 2-furyl.

44. The taxane of claim 20, wherein X₅ is -COOX₁₀ and X₁₀ is t-butyl, R₁₀ₐ is isobutenyl, and X₃ is 2-furyl, 3-furyl, 2-thienyl, or 3-thienyl.

45. The taxane of claim 20, wherein X₅ is -COX₁₀ and X₁₀ is 2-furyl or 2-thienyl, R₁₀ₐ is ethyl, cyclopropyl, or propyl, and X₃ is 2-furyl.

46. The taxane of claim 20, wherein X₅ is -COX₁₀ and X₁₀ is trans-propenyl, R₁₀ₐ is cyclopropyl, and X₃ is 3-thienyl.

47. The taxane of claim 20, wherein X₅ is -COX₁₀ and X₁₀ is phenyl, R₁₀ₐ is propyl, and X₃ is 2-furyl or 2-thienyl.

48. The taxane of claim 20, wherein X₅ is -COOX₁₀ and X₁₀ is tert-amyl, R₁₀ₐ is ethyl, and X₃ is 2-furyl.

49. A pharmaceutical composition comprising the taxane of any one of the previous claims and at least one pharmaceutically acceptable carrier.

50. The pharmaceutical composition of claim 49, for oral administration.

51. The use of a taxane according to any one of claims 1 to 48 for the manufacture of a pharmaceutical composition for inhibiting tumor growth in a mammal.

## Patentansprüche

1. Taxan gemäß der Formel: 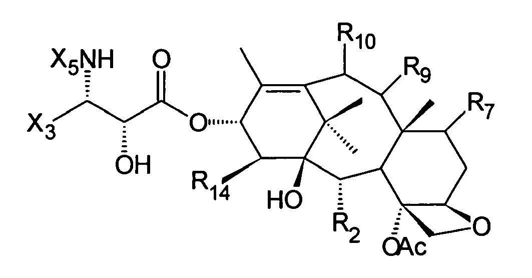 worin
R₂ Acyloxy ist;
R₇ ist Hydroxy;
R₉ ist Keto, Hydroxy oder Acyloxy;
R₁₀ ist RloaCOO-;
R₁₀ₐ ist ein Kohlenwasserstoffrest oder substituierter Kohlenwasserstoffrest, wobei der Kohlenwasserstoffrest oder substituierte Kohlenwasserstoffrest Kohlenstoffatome in den alpha- und beta-Positionen relativ zu dem Kohlenstoff enthält, an dem R₁₀ₐ ein Substituent ist;
R₁₄ ist Wasserstoff oder Hydroxy;
X₃ ist Heterozyklus;
X₅ ist -COX₁₀, -COOX₁₀, oder -CONHX₁₀;
X₁₀ ist Kohlenwasserstoffrest, substituierter Kohlenwasserstoffrest oder Heterozyklus; und
Ac ist Acetyl.

2. Taxan nach Anspruch 1, wobei R₁₀ₐ C₂ - C₈ Alkyl ist.

3. Taxan nach Anspruch 2, wobei X₃ 2-Furyl, 3-Furyl, 2-Thienyl, 3-Thienyl, 2-Pyridyl, 3-Pyridyl oder 4-Pyridyl ist.

4. Taxan nach Anspruch 2, wobei X₅ gleich -COX₁₀ und X₁₀ substituiertes oder unsubstituiertes Phenyl, 2-Furyl, 3-Furyl, 2-Thienyl, 3-Thienyl, 2-Pyridyl, 3-Pyridyl, 4-Pyridyl, C₁ - C₈ Alkyl, C₂ - C₈ Alkenyl oder C₂ - C₈ Alkinyl ist, oder X₅ ist -COOX₁₀ und X₁₀ ist substituiertes oder unsubstituiertes C₁ - C₈ Alkyl, C₂ - C₈ Alkenyl, oder C₂ - C₈ Alkinyl.

5. Taxan nach einem der Ansprüche 2 bis 4, wobei R₁₄ Wasserstoff ist.

6. Taxan nach einem der Ansprüche 2 bis 4, wobei R₂ Benzoyloxy ist.

7. Taxan nach einem der Ansprüche 2 bis 4, wobei R₁₄ Wasserstoff und R₉ Keto ist.

8. Taxan nach einem der Ansprüche 2 bis 4, wobei R₂ Benzoyloxy und R₉ Keto ist.

9. Taxan nach einem der Ansprüche 2 bis 4, wobei R₁₄ Wasserstoff und R₂ Benzoyloxy ist.

10. Taxan nach einem der Ansprüche 2 bis 4, wobei R₁₄ Wasserstoff, R₉ Keto und R₂ Benzoyloxy ist.

11. Taxan nach Anspruch 1, wobei R₁₀ₐ Ethyl ist.

12. Taxan nach Anspruch 11, wobei X₃ 2-Furyl, 3-Furyl, 2-Thienyl, 3-Thienyl, 2-Pyridyl, 3-Pyridyl oder 4-Pyridyl ist.

13. Taxan nach Anspruch 11, wobei X₅ gleich -COX₁₀ und X₁₀ substituiertes oder unsubstituiertes Phenyl, 2-Furyl, 3-Furyl, 2-Thienyl, 3-Thienyl, 2-Pyridyl, 3-Pyridyl, 4-Pyridyl, C₁ - C₈ Alkyl, C₂ - C₈ Alkenyl oder C₂ - C₈ Alkinyl ist, oder X₅ ist -COOX₁₀ und X₁₀ ist substituiertes oder unsubstituiertes C₁ - C₈ Alkyl, C₂ - C₈ Alkenyl oder C₂ - C₈ Alkinyl.

14. Taxan nach einem der Ansprüche 11 bis 13, wobei R₁₄ Wasserstoff ist.

15. Taxan nach einem der Ansprüche 11 bis 13, wobei R₂ Benzoyloxy ist.

16. Taxan nach einem der Ansprüche 11 bis 13, wobei R₁₄ Wasserstoff und R₉ Keto ist.

17. Taxan nach einem der Ansprüche 11 bis 13, wobei R₂ Benzoyloxy und R₉ Keto ist.

18. Taxan nach einem der Ansprüche 11 bis 13, wobei R₁₄ Wasserstoff und R₂ Benzoyloxy ist.

19. Taxan nach einem der Ansprüche 11 bis 13, wobei R₁₄ Wasserstoff, R₉ Keto und R₂ Benzoyloxy ist.

20. Taxan nach Anspruch 1 gemäß der Formel: 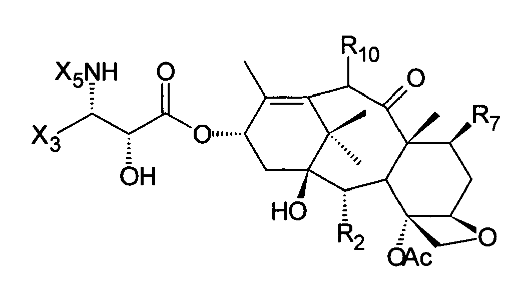 worin
R₂ Benzoyloxy ist;
R₇ ist Hydroxy;
R₁₀ ist R₁₀ₐCOO-;
X₃ ist Heterozyklus;
X₅ ist -COX₁₀, -COOX₁₀ oder -CONHX₁₀;
X₁₀ ist Kohlenwasserstoffrest, substituierter Kohlenwasserstoffrest oder Heterozyklus; und
R₁₀ₐ ist ein Kohlenwasserstoffrest oder substituierter Kohlenwasserstoffrest, wobei der Kohlenwasserstoffrest oder substituierte Kohlenwasserstoffrest Kohlenstoffatome in den alpha- und beta-Positionen relativ dem Kohlenstoffatom enthält, an dem R₁₀ₐ, ein Substituent ist; und
Ac ist Acetyl.

21. Taxan nach Anspruch 20, wobei X₃ 2-Furyl, 3-Furyl, 2-Thienyl, 3-Thienyl, 2-Pyridyl, 3-Pyridyl oder 4-Pyridyl ist.

22. Taxan nach Anspruch 20, wobei X₅ gleich -COX₁₀ und X₁₀ substituiertes oder unsubstituiertes Phenyl, 2-Furyl, 3-Furyl, 2-Thienyl, 3-Thienyl, 2-Pyridyl, 3-Pyridyl, 4-Pyridyl, C₁ - C₈ Alkyl, C₂ - C₈ Alkenyl oder C₂ - C₈ Alkinyl ist, oder X₅ ist -COOX₁₀ und X₁₀ ist substituiertes oder unsubstituiertes C₁ - C₈ Alkyl, C₂ - C₈ Alkenyl oder C₂ - C₈ Alkinyl.

23. Taxan nach einem der Ansprüche 20 bis 22, wobei X₃ Furyl oder Thienyl ist.

24. Taxan nach einem der Ansprüche 20 bis 22, wobei R₁₀ₐ Ethyl oder Propyl ist

25. Taxan nach Anspruch 20, wobei X₃ Furyl oder Thienyl ist, R₁₀ₐ ist Ethyl und X₅ ist -COX₁₀ und X₁₀ ist Phenyl, oder X₅ ist -COOX₁₀ und X₁₀ ist t-Butyl.

26. Taxan nach Anspruch 20, wobei X₃ 2-Furyl oder 2-Thienyl ist, R₁₀ₐ ist Ethyl, X₅ ist -COOX₁₀ und X₁₀ ist t-Butyl.

27. Taxan nach Anspruch 20, wobei X₅ -COOX₁₀ und X₁₀ t-Butyl ist, R₁₀ₐ ist Ethyl, Propyl, Isopropyl oder trans-Propenyl, und X₃ ist 2-Furyl oder 3-Furyl.

28. Taxan nach Anspruch 20, wobei X₅ -COOX₁₀ und X₁₀ t-Butyl ist, R₁₀ₐ ist Ethyl, Propyl, Isopropyl oder trans-Propenyl, und X₃ ist 2-Thienyl oder 3-Thienyl.

29. Taxan nach Anspruch 20, wobei X₅ -COX₁₀ und X₁₀ Phenyl ist, R₁₀ₐ ist Ethyl und X₃ ist 2-Pyridyl, 3-Pyridyl, 4-Pyridyl, 2-Furyl, 3-Furyl, 2-Thienyl oder 3-Thienyl.

30. Taxan nach Anspruch 20, wobei X₅ -COOX₁₀ und X₁₀ Ethyl ist, R₁₀ₐ ist Ethyl, Cyclopropyl oder Propyl, und X₃ ist 2-Thienyl oder 2-Furyl.

31. Taxan nach Anspruch 20, wobei X₅ -COX₁₀ und X₁₀ 2-Furyl oder 2-Thienyl ist, R₁₀ₐ ist Ethyl, Cyclopropyl oder Propyl, und X₃ ist 2-Thienyl.

32. Taxan nach Anspruch 20, wobei X₅ -COOX₁₀ und X₁₀ t-Butyl ist, R₁₀ₐ ist Cyclopropyl, und X₃ ist 2-Furyl, 3-Furyl, 2-Thienyl oder 3-Thienyl.

33. Taxan nach Anspruch 20, wobei X₅ -COOX₁₀ und X₁₀ Isobutyl ist, R₁₀ₐ ist Cyclopropyl oder Propyl, und X₃ ist 2-Furyl oder 3-Furyl.

34. Taxan nach Anspruch 20, wobei X₅ -COOX₁₀ und X₁₀ Isobutyl ist, R₁₀ₐ ist Cyclopropyl, Propyl oder Ethyl, und X₃ ist 2-Thienyl.

35. Taxan nach Anspruch 20, wobei X₅ -COX₁₀ und X₁₀ trans-Propenyl, Isobutenyl oder Butenyl ist, R₁₀ₐ ist Propyl, Cyclopropyl oder Ethyl, und X₃ ist 2-Thienyl.

36. Taxan nach Anspruch 20, wobei X₅ -COX₁₀ und X₁₀ Butenyl oder trans-Propenyl ist, R₁₀ₐ ist Propyl, Cyclopropyl oder Ethyl, und X₃ ist 2-Furyl.

37. Taxan nach Anspruch 20, wobei X₅ -COX₁₀ und X₁₀ trans-Propenyl ist, R₁₀ₐ ist n-Propyl, Cyclopropyl oder Ethyl, und X₃ ist 3-Furyl.

38. Taxan nach Anspruch 20, wobei X₅ -COX₁₀ und X₁₀ Phenyl ist, R₁₀ₐ ist Cyclopropyl, und X₃ ist 2-Pyridyl, 4-Pyridyl, 2-Furyl, 3-Furyl, 2-Thienyl oder 3-Thienyl.

39. Taxan nach Anspruch 20, wobei X₅ -COX₁₀ und X₁₀ n-Propyl ist, R₁₀ₐ ist Cyclopropyl, Ethyl oder Propyl, und X₃ ist 2-Furyl.

40. Taxan nach Anspruch 20, wobei X₅ -COX₁₀ und X₁₀ n-Propyl ist, R₁₀ₐ ist Cyclopropyl oder Propyl, und X₃ ist 2-Thienyl.

41. Taxan nach Anspruch 20, wobei X₅ -COOX₁₀ und X₁₀ Isobutyl ist, R₁₀, ist Propyl, und X₃ ist 2-Furyl.

42. Taxan nach Anspruch 20, wobei X₅ -COOX₁₀ und X₁₀ Isobutyl ist, R₁₀ₐ ist n-Propyl, Cyclopropyl oder Ethyl, und X₃ ist 3-Thienyl.

43. Taxan nach Anspruch 20, wobei X₅ -COOX₁₀ und X₁₀ Isopropyl ist, R₁₀ₐ ist Ethyl, Cyclopropyl oder Propyl, und X₃ ist 2-Thienyl oder 2-Furyl.

44. Taxan nach Anspruch 20, wobei X₅ -COOX₁₀ und X₁₀ t-Butyl ist, R₁₀ₐ ist Isobutenyl, und X₃ ist 2-Furyl, 3-Furyl, 2-Thienyl oder 3-Thienyl.

45. Taxan nach Anspruch 20, wobei X₅ -COX₁₀ und X₁₀ 2-Furyl oder 2-Thienyl ist, R₁₀ₐ ist Ethyl, Cyclopropyl oder Propyl, und X₃ ist 2-Furyl.

46. Taxan nach Anspruch 20, wobei X₅ -COX₁₀ und X₁₀ trans-Propenyl ist, R₁₀ₐ ist Cyclopropyl, und X₃ ist 3-Thienyl.

47. Taxan nach Anspruch 20, wobei X₅ -COX₁₀ und X₁₀ Phenyl ist, R₁₀ₐ ist Propyl, und X₃ ist 2-Furyl oder 2-Thienyl.

48. Taxan nach Anspruch 20, wobei X₅ -COOX₁₀ und X₁₀ tert-Amyl ist, R₁₀ₐ ist Ethyl, und X₃ ist 2-Furyl.

49. Pharmazeutische Zusammensetzung umfassend das Taxan nach einem der vorherigen Ansprüche und mindestens einen pharmazeutisch akzeptablen Träger.

50. Pharmazeutische Zusammensetzung nach Anspruch 49 für die orale Verabreichung.

51. Verwendung eines Taxans gemäß einem der Ansprüche 1 bis 48, zur Herstellung einer pharmazeutischen Zusammensetzung zur Hemmung des Tumorwachstums bei einem Säuger.

## Revendications

1. Taxane de formule 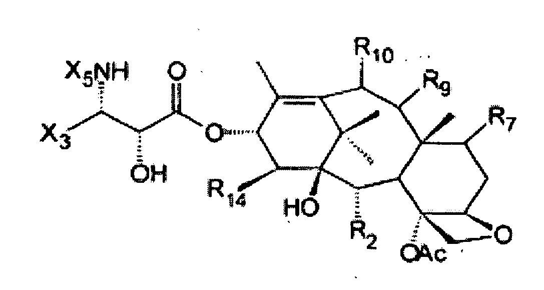
R₂ représente un groupe acyloxy ;
R₇ représente un groupe hydroxy ;
R₉ représente un groupe céto, hydroxy ou acyloxy ;
R₁₀ représente R₁₀ₐCOO- ;
R₁₀ₐ représente un groupe hydrocarbyle ou hydrocarbyle substitué, ledit groupe hydrocarbyle ou hydrocarbyle substitué contenant des atomes de carbone aux positions alpha et bêta par rapport à l'atome de carbone dont R₁₀ₐ est un substituant ;
R₁₄ représente un hydrogène ou un groupe hydroxy ;
X₃ représente un groupe hétérocyclo ;
X₅ représente -COX₁₀, -COOX₁₀ ou -CONHX₁₀ ;
X₁₀ représente un groupe hydrocarbyle, hydrocarbyle substitué ou hétérocyclo ; et
Ac représente un groupe acétyle.

2. Taxane de la revendication 1, dans lequel R₁₀ₐ représente un groupe alkyle C₂-C₈.

3. Taxane de la revendication 2, dans lequel X₃ représente un groupe 2-furyle, 3-furyle, 2-thiényle, 3-thiényle, 2-pyridyle, 3-pyridyle ou 4-pyridyle.

4. Taxane de la revendication 2, dans lequel X₅ représente -COX₁₀ et X₁₀ représente un groupe phényle, 2-furyle, 3-furyle, 2-thiényle, 3-thiényle, 2-pyridyle, 3-pyridyle, 4-pyridyle, alkyle C₁-C₈, alkényle C₂-C₈, ou alkynyle C₂-C₈, substitué ou non substitué, ou bien X₅ représente -COOX₁₀ et X₁₀ représente un groupe alkyle C₁-C₈, alkényle C₂-C₈, ou alkynyle C₂-C₈, substitué ou non substitué.

5. Taxane de l'une des revendications 2 à 4, dans lequel R₁₄ représente un hydrogène.

6. Taxane de l'une des revendications 2 à 4, dans lequel R₂ représente un groupe benzoyloxy.

7. Taxane de l'une des revendications 2 à 4, dans lequel R₁₄ représente un hydrogène et R₉ représente un groupe céto.

8. Taxane de l'une des revendications 2 à 4, dans lequel R₂ représente un groupe benzoyloxy et R₉ représente un groupe céto.

9. Taxane de l'une des revendications 2 à 4, dans lequel R₁₄ représente un hydrogène et R₂ représente un groupe benzoyloxy.

10. Taxane de l'une des revendications 2 à 4, dans lequel R₁₄ représente un hydrogène, R₉ représente un groupe céto et R₂ représente un groupe benzoyloxy.

11. Taxane de la revendication 1, dans lequel R₁₀ₐ représente un groupe éthyle.

12. Taxane de la revendication 11, dans lequel X₃ représente un groupe 2-furyle, 3-furyle, 2-thiényle, 3-thiényle, 2-pyridyle, 3-pyridyle ou 4-pyridyle.

13. Taxane de la revendication 11, dans lequel X₅ représente -COX₁₀ et X₁₀ représente un groupe phényle, 2-furyle, 3-furyle, 2-thiényle, 3-thiényle, 2-pyridyle, 3-pyridyle, 4-pyridyle, alkyle C₁-C₈, alkényle C₂-C₈, ou alkynyle C₂-C₈, substitué ou non substitué, ou bien X₅ représente -COOX₁₀ et X₁₀ représente un groupe alkyle C₁-C₈, alkényle C₂-C₈, ou alkynyle C₂-C₈, substitué ou non substitué.

14. Taxane de l'une des revendications 11 à 13, dans lequel R₁₄ représente un hydrogène.

15. Taxane de l'une des revendications 11 à 13, dans lequel R₂ représente un groupe benzoyloxy.

16. Taxane de l'une des revendications 11 à 13, dans lequel R₁₄ représente un hydrogène et R₉ représente un groupe céto.

17. Taxane de l'une des revendications 11 à 13, dans lequel R₂ représente un groupe benzoyloxy et R₉ représente un groupe céto.

18. Taxane de l'une des revendications 11 à 13, dans lequel R₁₄ représente un hydrogène et R₂ représente un groupe benzoyloxy.

19. Taxane de l'une des revendications 11 à 13, dans lequel R₁₄ représente un hydrogène, R₉ représente un groupe céto et R₂ représente un groupe benzoyloxy.

20. Taxane de la revendication 1 de formule 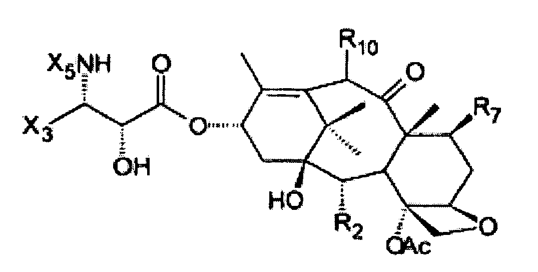
R₂ représente un groupe benzoyloxy ; .
R₇ représente un groupe hydroxy ;
R₁₀ représente R₁₀ₐCOO- ;
X₃ représente un groupe hétérocyclo ;
X₅ représente -COX₁₀, -COOX₁₀ ou -CONHX₁₀ ;
X₁₀ représente un groupe hydrocarbyle, hydrocarbyle substitué ou hétérocyclo ; et
R₁₀ₐ représente un groupe hydrocarbyle ou hydrocarbyle substitué, ledit groupe hydrocarbyle ou hydrocarbyle substitué contenant des atomes de carbone aux positions alpha et bêta par rapport à l'atome de carbone dont R₁₀ₐ est un substituant ; et
Ac représente un groupe acétyle.

21. Taxane de la revendication 20, dans lequel X₃ représente un groupe 2-furyle, 3-furyle, 2-thiényle, 3-thiényle, 2-pyridyle, 3-pyridyle ou 4-pyridyle.

22. Taxane de la revendication 20, dans lequel X₅ représente -COX₁₀ et X₁₀ représente un groupe phényle, 2-furyle, 3-furyle, 2-thiényle, 3-thiényle, 2-pyridyle, 3-pyridyle, 4-pyridyle, alkyle C₁-C₈, alkényle C₂-C₈, ou alkynyle C₂-C₈, substitué ou non substitué, ou bien X₅ représente -COOX₁₀ et X₁₀ représente un groupe alkyle C₁-C₈, alkényle C₂-C₈, ou alkynyle C₂-C₈, substitué ou non substitué.

23. Taxane de l'une des revendications 20 à 22, dans lequel X₃ représente un groupe furyle ou thiényle.

24. Taxane de l'une des revendications 20 à 22, dans lequel R₁₀ₐ, représente un groupe éthyle ou propyle.

25. Taxane de la revendication 20, dans lequel X₃ représente un groupe furyle ou thiényle, R₁₀ₐ représente un groupe éthyle et X₅ représente -COX₁₀ et X₁₀ représente un groupe phényle, ou bien X₅ représente -COOX₁₀ et X₁₀ représente un groupe t-butyle.

26. Taxane de la revendication 20, dans lequel X₃ représente un groupe 2-furyle ou 2-thiényle, R₁₀ₐ représente un groupe éthyle, X₅ représente -COOX₁₀ et X₁₀ représente un groupe t-butyle.

27. Taxane de la revendication 20, dans lequel X₅ représente -COOX₁₀ et X₁₀ représente un groupe t-butyle, R₁₀ₐ représente un groupe éthyle, propyle, isopropyle ou trans-propényle et X₃ représente un groupe 2-furyle ou 3-furyle.

28. Taxane de la revendication 20, dans lequel X₅ représente -COOX₁₀ et X₁₀ représente un groupe t-butyle, R₁₀ₐ représente un groupe éthyle, propyle, isopropyle ou trans-propényle et X₃ représente un groupe 2-thiényle ou 3-thiényle.

29. Taxane de la revendication 20, dans lequel X₅ représente -COX₁₀ et X₁₀ représente un groupe phényle, R₁₀ₐ représente un groupe éthyle et X₃ représente un groupe 2-pyridyle, 3-pyridyle, 4-pyridyle, 2-furyle, 3-furyle, 2-thiényle ou 3-thiényle.

30. Taxane de la revendication 20, dans lequel X₅ représente -COOX₁₀ et X₁₀ représente un groupe éthyle, R₁₀ₐ, représente un groupe éthyle, cyclopropyle ou propyle et X₃ représente un groupe 2-thiényle ou 2-furyle.

31. Taxane de la revendication 20, dans lequel X₅ représente -COX₁₀ et X₁₀ représente un groupe 2-furyle ou 2-thiényle, R₁₀ₐ représente un groupe éthyle, cyclopropyle ou propyle et X₃ représente un groupe 2-thiényle.

32. Taxane de la revendication 20, dans lequel X₅ représente -COOX₁₀ et X₁₀ représente un groupe t-butyle, R₁₀ₐ représente un groupe cyclopropyle et X₃ représente un groupe 2-furyle, 3-furyle, 2-thiényle ou 3-thiényle.

33. Taxane de la revendication 20, dans lequel X₅ représente -COOX₁₀ et X₁₀ représente un groupe isobutyle, R₁₀ₐ représente un groupe cyclopropyle ou propyle et X₃ représente un groupe 2-furyle ou 3-furyle.

34. Taxane de la revendication 20, dans lequel X₅ représente -COOX₁₀ et X₁₀ représente un groupe isobutyle, R₁₀ₐ représente un groupe cyclopropyle, propyle ou éthyle et X₃ représente un groupe 2-thiényle.

35. Taxane de la revendication 20, dans lequel X₅ représente -COX₁₀ et X₁₀ représente un groupe trans-propényle, isobutényle ou butényle, R₁₀ₐ représente un groupe propyle, cyclopropyle ou éthyle et X₃ représente un groupe 2-thiényle.

36. Taxane de la revendication 20, dans lequel X₅ représente -COX₁₀ et X₁₀ représente un groupe butényle ou trans-propényle, R₁₀ₐ représente un groupe propyle, cyclopropyle ou éthyle et X₃ représente un groupe 2-furyle.

37. Taxane de la revendication 20, dans lequel X₅ représente -COX₁₀ et X₁₀ représente un groupe trans-propényle, R₁₀ₐ représente un groupe n-propyle, cyclopropyle ou éthyle et X₃ représente un groupe 3-furyle.

38. Taxane de la revendication 20, dans lequel X₅ représente -COX₁₀ et X₁₀ représente un groupe phényle, R₁₀ₐ représente un groupe cyclopropyle et X₃ représente un groupe 2-pyridyle, 4-pyridyle, 2-furyle, 3-furyle, 2-thiényle ou 3-thiényle.

39. Taxane de la revendication 20, dans lequel X₅ représente -COX₁₀ et X₁₀ représente un groupe n-propyle, R₁₀ₐ représente un groupe cyclopropyle, éthyle ou propyle et X₃ représente un groupe 2-furyle.

40. Taxane de la revendication 20, dans lequel X₅ représente -COX₁₀ et X₁₀ représente un groupe n-propyle, R₁₀ₐ représente un groupe cyclopropyle ou propyle et X₃ représente un groupe 2-thiényle.

41. Taxane de la revendication 20, dans lequel X₅ représente -COOX₁₀ et X₁₀ représente un groupe isobutyle, R₁₀ₐ représente un groupe propyle et X₃ représente un groupe 2-furyle.

42. Taxane de la revendication 20, dans lequel X₅ représente -COOX₁₀ et X₁₀ représente un groupe isobutyle, R₁₀ₐ représente un groupe n-propyle, cyclopropyle ou éthyle et X₃ représente un groupe 3-thiényle.

43. Taxane de la revendication 20, dans lequel X₅ représente -COOX₁₀ et X₁₀ représente un groupe isopropyle, R₁₀ₐ représente un groupe éthyle, cyclopropyle ou propyle et X₃ représente un groupe 2-thiényle ou 2-furyle.

44. Taxane de la revendication 20, dans lequel X₅ représente -COOX₁₀ et X₁₀ représente un groupe t-butyle, R₁₀ₐ représente un groupe isobutényle et X₃ représente un groupe 2-furyle, 3-furyle, 2-thiényle ou 3-thiényle.

45. Taxane de la revendication 20, dans lequel X₅ représente -COX₁₀ et X₁₀ représente un groupe 2-furyle ou 2-thiényle, R₁₀ₐ représente un groupe éthyle, cyclopropyle ou propyle et X₃ représente un groupe 2-furyle.

46. Taxane de la revendication 20, dans lequel X₅ représente -COX₁₀ et X₁₀ représente un groupe trans-propényle, R₁₀ₐ représente un groupe cyclopropyle et X₃ représente un groupe 3-thiényle.

47. Taxane de la revendication 20, dans lequel X₅ représente -COX₁₀ et X₁₀ représente un groupe phényle, R₁₀ₐ représente un groupe propyle et X₃ représente un groupe 2-furyle ou 2-thiényle.

48. Taxane de la revendication 20, dans lequel X₅ représente -COOX₁₀ et X₁₀ représente un groupe tert-amyle, R₁₀ₐ représente un groupe éthyle et X₃ représente un groupe 2-furyle.

49. Composition pharmaceutique contenant le taxane de l'une des revendications précédentes et au moins un porteur pharmaceutiquement acceptable.

50. Composition pharmaceutique de la revendication 49 pour une administration orale.

51. Utilisation d'un taxane de l'une des revendications 1 à 48 pour la fabrication d'une composition pharmaceutique permettant d'inhiber la croissance d'une tumeur chez un mammifère.
